(19) Europäisches Patentamt
European Patent Office
Office européen des brevets

(11) **EP 1 372 598 B1**

(12) **FASCICULE DE BREVET EUROPEEN**

(45) Date de publication et mention de la délivrance du brevet:
**17.08.2011 Bulletin 2011/33**

(21) Numéro de dépôt: **02720089.8**

(22) Date de dépôt: **02.04.2002**

(51) Int Cl.:
***A61K 8/97*** *(2006.01)* ***A61Q 19/08*** *(2006.01)*

(86) Numéro de dépôt international:
**PCT/FR2002/001129**

(87) Numéro de publication internationale:
**WO 2002/080876 (17.10.2002 Gazette 2002/42)**

(54) **UTILISATION D'UN EXTRAIT D'ALGUE PHAEDOCTYLUM POUR FAVORISER L'ACTIVITE DU PROTEASOME DES CELLULES DE LA PEAU**

VERWENDUNG VON EINEM PHAEODACTYLUM-ALGEN-EXTRAKT ZUR FÖRDERUNG DER HAUTZELLEN-PROTEASOM-AKTIVITÄT

USE OF A PHAEODACTYLUM ALGAE EXTRACT TO PROMOTE THE PROTEASOME ACTIVITY OF SKIN CELLS

(84) Etats contractants désignés:
**AT BE CH CY DE DK ES FI FR GB GR IE IT LI LU MC NL PT SE TR**

(30) Priorité: **03.04.2001 FR 0104535**

(43) Date de publication de la demande:
**02.01.2004 Bulletin 2004/01**

(73) Titulaire: **LVMH RECHERCHE**
**45800 St. Jean de Braye (FR)**

(72) Inventeurs:
- **NIZARD, Carine**
  **F-94200 Ivry Sur Seine (FR)**
- **FRIGUET, Bertrand**
  **F-75013 Paris (FR)**
- **MOREAU, Marielle**
  **F-78770 Marcq (FR)**
- **BULTEAU, Anne-Laure**
  **F-75007 Paris (FR)**
- **SAUNOIS, Alex**
  **45240 Menestreau en Villette (FR)**

(74) Mandataire: **Portal, Gérard**
**Cabinet Beau de Loménie**
**158, rue de l'Université**
**75340 Paris Cedex 07 (FR)**

(56) Documents cités:
**EP-A- 0 646 647** **EP-A- 0 775 449**

- **DATABASE CAPLUS [en ligne] CHEMICAL ABSTRACTS SERVICE, COLUMBUS, OHIO, US; retrieved from STN Database accession no. 1995: 890657 XP002187712 & JP 07 224278 A (SANGYO SOZO KENKYUSHO) 22 août 1995 (1995-08-22)**
- **DATABASE WPI Week 198504 Derwent Publications Ltd., London, GB; AN 1985-021129 XP002187713 & JP 59 215386 A (IND RES INST OF JAPAN)**
- **PATENT ABSTRACTS OF JAPAN vol. 009, no. 071 (C-272) & JP 59 204128 A (KOUGIYOU KAIHATSU KENKYUSHO KK)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 372 598 B1

Printed by Jouve, 75001 PARIS (FR)

## Description

L'invention concerne essentiellement l'utilisation d'un extrait de l'algue Phaeodactylum, en particulier de l'algue Phaeodactylum tricornutum, comme agent cosmétique favorisant l'activité du protéasome des cellules de la peau, en particulier des kératinocytes, des fibroblastes ou des mélanocytes, de préférence humains ainsi qu'une composition cosmétique le contenant, et un procédé de soin cosmétique.

L'invention concerne encore essentiellement l'utilisation de l'extrait de l'algue Phaeodactylum, en particulier Phaeodactylum tricornutum, pour la fabrication d'une composition cosmétique destinée à protéger la peau contre les effets néfastes d'une exposition aux UV, ou à prévenir et/ou retarder l'apparition des effets du vieillissement cutané.

La recherche de moyens de lutte contre le vieillissement de l'homme mobilise de nos jours un grand nombre de chercheurs. C'est en particulier le cas dans le domaine de la cosmétique où l'on cherche à lutter contre, ou au moins à ralentir, l'apparition des effets apparents du vieillissement cutané, tels que les rides, la perte d'élasticité ou de couleur de la peau, etc.

Les expositions plus ou moins prolongées aux radiations solaires, et principalement aux rayonnements ultraviolets sont aujourd'hui bien connues pour leurs conséquences néfastes, à moyen ou long terme. Bien que la pigmentation de la peau protège contre les UVA (longueur d'onde 320 à 400 nanomètres) de manière immédiate mais aussi retardée en ce qui concerne les UVB (longueur d'onde de 290 à 320 nanomètres), une exposition prolongée aux rayonnements ultraviolets peut provoquer un érythème actinique, ou de l'élastose solaire, accélérer l'apparition des effets du vieillissement cutané tel que les rides, et parfois même conduire à la formation d'un cancer cutané. Il est admis que les UVA, dont la majorité traverse l'épiderme, entraînent la production d'espèces oxydantes comme les radicaux libres, des formes réactives de l'oxygène, qui réagissent à divers niveaux dans la peau en produisant des altérations des cellules cutanées, et en particulier des kératinocytes, des fibroblastes ou des mélanocytes. Il en résulte des manifestations inflammatoires ou l'apparition d'un vieillissement actinique, en particulier sous forme de rides.

Il est admis depuis longtemps déjà que la protection par voie topique de la peau contre les rayonnements UV est mise en oeuvre de manière classique par l'emploi de filtres physiques et/ou de filtres chimiques dans les produits solaires ou les produits destinés à lutter contre le vieillissement.

On sait d'autre part d'après une publication de Friguet B. et al. dans Ann N.Y. Acad. Sci. 2000 ; 908 : 143-54 intitulée "Protein dégradation by the proteasome and its implication in aging" que le protéasome est un complexe protéique multicatalytique connu pour son rôle au niveau de l'assainissement cellulaire et dont la fonction principale est d'effectuer dans les cellules l'élimination des protéines endommagées par oxydation. Au cours de cette élimination se forme des peptides qui sont ensuite métabolisés par la cellule. Ainsi, le protéasome débarrasse la cellule d'éléments inutiles qui entravent son fonctionnement optimal en s'accumulant, comme décrit par Petropoulos, Friguet et al. dans Journal of Gerontol. Biol. Sci., 2000 ; 55A, N°5 B220-B227 intitulé "Increase of oxidatively modified protein is associated with a decrease of proteasome activity and content in aging epidermal cells".

L'abrégé XP-002187712 de la base de données CAPLUS cite l'abrégé de la demande japonaise JP 07 224 278 A de 1995, qui décrit des anti-oxydants contenant la fucoxanthine, leurs utilisations dans des produits alimentaires cosmétiques et pharmaceutiques. La fucoxanthine peut être extraite à partir de l'algue *Phaeodactylum tricornutum.*

L'abrégé XP-002187713 DERWENT cite la demande japonaise JP 59 215386, qui décrit également un anti-oxydant produit à partir de diatomée *Phaeodactylum tricornutum.* Cette action anti-oxydante est indiquée comme étant supérieure à la vitamine E et peut être utilisée pour prévenir la dénaturation des produits pharmaceutiques ou alimentaires.

L'abrégé de Patent Abstracts of Japan, volume 009, N°071, cite la demande japonaise JP 59 204128, qui indique l'extraction de l'acide $\omega$-3-eicosapentaenoique comme agent anti-artériosclérose.

Le document EP 0 646 647 décrit un procédé de préparation de boues thermales utilisables en thermalisme, en cosmétologique et en usage vétérinaire. Ces boues thermales apparaissent être utilisées pour traiter la peau, et notamment pour agir contre les rhumatismes (page 1, lignes 3-5), même si une utilisation en cosmétologie est envisagée (revendication 25).

Le document EP 0 775 449 est relatif à la production d'oeufs de volailles avec une teneur élevée en acide gras hautement insaturé, notamment un acide arachidonique, et éventuellement un acide docosahéxaénoique. Il est indiqué que des microorganismes du genre *Phaeodactylum* permettent de produire un tel acide docosahéxaénoique.

La présente invention a pour but principal de résoudre le nouveau problème technique consistant en la fourniture d'un nouvel agent cosmétique capable de favoriser l'activité du protéasome des cellules de la peau, en particulier des kératinocytes, des fibroblastes ou des mélanocytes, de préférence humains.

La présente invention a encore pour but principal de résoudre le nouveau problème technique consistant en l'utilisation d'une nouvelle composition capable de protéger la peau contre les effets néfastes d'une exposition aux UV, ou de prévenir et/ou retarder l'apparition des effets du vieillissement cutané.

La présente invention a encore pour but de résoudre le problème technique consistant en la fourniture d'un moyen complémentaire de celui de la protection par des filtres classiques UV notamment en secondant leur action en intervenant au niveau du bon fonctionnement cellulaire, en favorisant la détoxication des cellules.

L'ensemble de ces problèmes techniques est résolu pour la première fois par la présente invention d'une façon simple, peu coûteuse, fiable, utilisable à l'échelle industrielle en cosmétique.

Ainsi, selon un premier aspect, la présente invention concerne l'utilisation d'un extrait de l'algue Phaeodactylum, en particulier de l'algue Phaeodactylum tricornutum, comme agent cosmétique favorisant l'activité du protéasome des cellules de la peau, en particulier des kératinocytes, des fibroblastes ou des mélanocytes, de préférence humains.

Selon un deuxième aspect, la présente invention concerne encore une composition comprenant un extrait de l'algue Phaeodactylum, en particulier de l'algue Phaeodactylum Tricornutum, pour protéger la peau contre les effets néfastes d'une exposition aux UV, ou prévenir et/ou retarder le vieillissement cutané.

Un procédé de soin de la peau; comprend l'application topique sur les zones concernées de la peau d'une personne en ayant besoin, d'une quantité efficace d'un extrait de l'algue Phaeodactylum, en particulier de l'algue Phaeodactylum Tricornutum, en vue d'obtenir un effet préventif ou un effet réparateur selon que l'application topique est effectuée avant ou après exposition aux rayonnements UV. L'effet global ainsi obtenu est un effet anti-vieillissement sur lesdites zones de la peau, notamment en améliorant la fermeté cutanée, l'élasticité de la peau, en retardant l'apparition des rides ou en diminuant leur profondeur.

Dans le cadre de l'un quelconque des aspects de la présente invention, l'extrait de l'algue Phaeodactylum, en particulier de l'algue Phaeodactylum Tricornutum, peut être présent dans une composition comprenant de 0,01 % à 10 % environ, et en particulier de 0,1 % à 5 % environ, par rapport au poids total de la composition finale.

Il est rappelé que l'algue Phaeodactylum, en particulier l'algue Phaeodactylum Tricornutum, est une algue diatomée qui provient des climats tempérés. Cette algue est connue pour son taux de croissance élevé, ce qui permet à certaines industries comme l'agro-alimentaire de l'utiliser comme source rapide de lipides qui iront enrichir différents produits ou bien comme nourriture pour l'aquaculture.

Selon un premier mode de réalisation, l'extrait est obtenu par extraction au moyen d'un solvant d'extraction polaire et/ou d'un solvant d'extraction apolaire.

L'extrait de l'algue Phaeodactylum, en particulier de l'algue Phaeodactylum tricornutum peut être, en particulier, obtenu par extraction au moyen d'un solvant d'extraction polaire et/ou d'un solvant d'extraction apolaire, en particulier un alcool en $C_1$-$C_6$ ou un mélange hydro-alcoolique, un polyalcool en $C_2$-$C_6$ tel que l'éthylène glycol, un solvant chloré tel que le chloroforme ou le dichlorométhane, un ester en $C_3$-$C_6$ d'acide organique tel qu'acétate d'éthyle, un alcane en $C_6$-$C_{10}$ tel que l'heptane, un éther en $C_5$-$C_8$ comme par exemple l'éther diisopropylique.

Selon une variante avantageuse de l'invention, cet extrait est un extrait obtenu par extraction de l'algue Phaeodactylum, en particulier de l'algue Phaeodactylum Tricornutum, avec un alcool ou un mélange eau/alcool éventuellement alcalinisé, ledit alcool étant choisi parmi le groupe consistant de l'isopropanol, de l'éthanol, et du méthanol.

On choisira avantageusement comme alcool l'isopropanol ou l'éthanol.

D'une manière générale avant toute opération d'extraction, l'algue est avantageusement congelée. De préférence la congélation est réalisée à une température située entre - 40°C et - 20°C environ et pendant une durée comprise de préférence entre 1 et 7 jours environ. Cette étape préalable est avantageusement utilisée pour réaliser un choc thermique par contact avec le futur solvant d'extraction afin de faciliter la décantation de la silice (issue du squelette des cellules de l'algue). L'algue est ensuite mise en contact avec le solvant d'extraction.

Selon une variante de réalisation avantageuse, l'algue congelée est plongée directement dans le solvant d'extraction chauffé.

On réalise également avantageusement une macération de l'algue dans le solvant d'extraction à température ambiante.

Selon une variante de réalisation avantageuse, on réalise une macération de l'algue à température ambiante et de préférence pendant une durée comprise entre 5 minutes et 80 minutes environ, et de préférence encore, pendant une durée comprise entre 20 minutes et 40 minutes environ.

Selon encore une variante de réalisation avantageuse, l'extraction est réalisée sous reflux.

Selon encore une autre variante de réalisation avantageuse, l'extraction peut-être réalisée sous atmosphère inerte, de préférence sous atmosphère saturée en azote. Ceci permet d'éviter en particulier une dégradation oxydative prononcée des molécules actives.

Le conditionnement de cet extrait est réalisé avantageusement sous gaz inerte tel que de l'azote, des antioxydants pouvant être également ajoutés afin de protéger les molécules actives.

Selon une variante de réalisation avantageuse, la quantité de solvant d'extraction utilisée est comprise entre 0,1 litre et 20 litres environ, de préférence comprise entre 2 litres et 10 litres environ, pour une quantité de 100 g d'algue, exprimée en poids sec d'algue.

Selon encore une variante avantageuse, dans le cas d'une extraction avec un alcool ou un mélange hydroalcoolique alcalinisé, l'extrait de l'algue précité est obtenu après la succession des étapes suivantes dont certaines sont décrites ci-dessus :

a) l'algue est congelée comme décrit précédemment puis plongée dans le solvant d'extraction

b) Une macération de l'algue est effectuée
c) le solvant d'extraction est alcalinisé jusqu'à un pH compris entre 10 et 14, de préférence à un pH égal à 13, par exemple avec une solution aqueuse d'hydroxyde de sodium ou avec une solution aqueuse d'hydroxyde de potassium,
d) les insolubles sont éliminés de la phase alcoolique ou hydro-alcoolique,
e) de l'eau distillée est ajoutée à la phase alcoolique ou hydro-alcoolique,
f) la solution hydro-alcoolique ainsi obtenue est lavée par un procédé liquide/liquide avec un solvant apolaire non miscible avec la phase alcoolique ou hydro-alcoolique, tel que par exemple l'heptane, l'hexane ou le cyclohexane,
g) la phase contenant le solvant apolaire est éliminée,
h) la phase hydro-alcoolique récupérée après élimination de la phase contenant le solvant apolaire, est acidifiée jusqu'à un pH compris entre 1 et 3, de préférence à un pH égal à 2, par exemple avec une solution aqueuse d'acide sulfurique ou avec une solution aqueuse d'acide chlorhydrique,
i) la solution obtenue après acidification subit une extraction liquide-liquide avec un solvant apolaire non miscible avec la phase alcoolique ou hydro-alcoolique, tel que par exemple l'heptane, l'hexane ou le cyclohexane,
j) la phase hydro-alcoolique est ensuite éliminée,
k) la phase contenant le solvant apolaire récupérée après élimination de la phase hydro-alcoolique subit une évaporation afin d'obtenir une huile exempte de solvant apolaire, cette huile étant l'extrait recherché selon l'invention.

L'emploi d'un alcool alcalinisé, puis acidifié permet d'obtenir un extrait aux caractéristiques visuelles et olfactives acceptables dans des compositions cosmétiques (couleur jaune, et odeur acceptable).

Selon un deuxième mode de réalisation avantageux de l'invention, l'extrait de l'algue précité est obtenu par extraction de l'algue au $CO_2$ supercritique. L'usage de ce solvant particulier implique que l'algue ait été au préalable lyophilisée.

D'autres caractéristiques, buts et avantages de la présente invention apparaîtront clairement à la lumière de la description explicative qui va suivre faite en référence à plusieurs exemples de réalisation de l'invention, ainsi qu'à des essais d'activité comparatifs, et des exemples de formulation de compositions cosmétiques, donnés simplement à titre d'illustration et qui ne sauraient donc en aucune façon limiter la portée de l'invention.

Dans les exemples, sauf indication contraire, les proportions données sont exprimées en pourcentage en poids. La température est en degré Celsius et la pression est la pression atmosphérique.

## Exemple I Obtention d'extraits de l'algue Phaeodactylum, en particulier de l'algue Phaeodactylum tricornutum.

### 1.1- Extraction avec un solvant polaire, tel que l'isopropanol (IPA) suivant un premier procédé

Selon le mode préféré du procédé, l'ensemble de l'extraction est réalisé sous atmosphère inerte (saturation en azote) afin d'éviter une dégradation prononcée des molécules actives.

Dans cet exemple, on utilise 250 kg de biomasse (Phaeodactylum Tricornutum).

Cette biomasse, qui est congelée à -20°C, puis plongée dans de l'isopropanol (IPA) porté au reflux à 80-83°C, et ce, sous agitation. Le choc thermique permet de faciliter la décantation de la silice (issue du squelette des cellules de l'algue).

La quantité de solvant utilisée est de 10 litres d'IPA pour 1 litre d'eau contenue dans la biomasse.Ainsi, pour un pourcentage de matière sèche de 30%, les 250 kg précités de biomasse se répartissent comme suit en une quantité de matière sèche de 75 kg et 175 kg d'eau. La quantité d'IPA utilisée est ici de (1750) kg.

L'ensemble (biomasse + IPA) est maintenu au reflux pendant une demi-heure sous agitation à environ 80°C, avant d'être refroidi vers 50°C.

Après le refroidissement de la biomasse et de l'IPA vers 50°C, l'ensemble est transféré dans un filtre de type GUEDU afin de réaliser la séparation biomasse épuisée / extrait d'algue sotubilisé dans l'IPA.

L'extrait est concentré dans un réacteur batch (facteur de concentration = 71,5). L'extrait concentré présente un aspect huileux.

Cet extrait huileux est ensuite repris dans de l'IPA à froid à raison de 10 kg de solvant pour 1 kg d'huile. L'agitation est prolongée pendant 20 minutes. Le jus est ensuite filtré (ceci permet d'éliminer la boue collante résiduelle).

Un traitement de décoloration et de désodorisation est réalisé en deux batchs dans un réacteur Schott de 80 litres et dure 30 minutes à température ambiante par addition de zéolithe et de charbon actif. La quantité de zéolithe (ABSENT 2000, fournisseur UOP) ajoutée est de 0,94 kg et celle de charbon actif (CXV, fournisseur CECA) est de 1,6 kg. Le rapport charbon sur zéolithe est de 1,7.

La zéolithe et le charbon sont ensuite éliminés par filtration sur papier.

Des antioxydants (DL $\alpha$-tocophérol à 0,05% de concentration finale en poids et palmitate d'ascorbyle à 0,05% de concentration finale en poids) sont incorporés par une solution mère dans de l'IPA.

Le filtrat contenant les antioxydants est ensuite concentré en batch, sous gaz inerte tel que de l'azote jusqu'à l'obtention d'une huile de couleur brune.

Cette huile sera nommée ci-après : l'extrait E1 selon l'invention de l'algue Phaeodactylum Tricornutum.

1.2 - Extraction suivant un second procédé en deux étapes, solvant polaire/éthanol, puis extraction liquide/liquide, solvant polaire (hydroéthanolique), solvant apolaire (heptane)

L'extraction débute par une dispersion de 49,8 kg de masse sèche congelée issue de 250 kg de biomasse (Phaeodactylum Tricornutum), soit environ 20% en masse sèche dans 539 kg d'éthanol anhydre à 96%, alcalinisé par 9 kg de solution aqueuse sodique à 30,5%. Après une macération de 30 minutes au reflux de l'éthanol et sous atmosphère azotée, l'ensemble est refroidi à 18°C.

Les insolubles sont alors séparés par essorage sous azote et sont éliminés.

Les 573,9 kg de filtrat sont additionnés de 151 kg d'eau distillée. Cette phase hydro-alcoolique est agitée lentement pendant 10 minutes pour être ensuite lavée grâce à un procédé liquide/liquide avec 162 kg d'heptane. L'épiphase heptanique de la partition liquide/liquide est éliminée. L'hypophase est récupérée car elle contient les acides gras sous forme saline, en raison de l'alcalinisation pratiquée au début de l'extraction. L'opération de lavage heptanique est répétée deux autres fois et l'hypophase est récupérée systématiquement.

Les 720 kg d'hypophase ainsi obtenus sont acidifiés par ajout de 2,8 kg d'acide sulfurique pour amener le pH à une valeur de 2,2 et obtenir ainsi les acides gras sous forme acide. L'ensemble de la solution est agité 10 minutes sous azote pour être ensuite soumis à une extraction liquide/liquide avec un solvant apolaire, ledit solvant apolaire étant constitué ici par une fraction de 158 kg d'heptane. L'opération de lavage à l'heptane est répétée cinq fois pour récupérer au total 697 kg de phase heptanique issus des cinq fractions contenant les acides gras libres. Cette phase évaporée à sec à l'évaporateur rotatif puis par distillation moléculaire fournit l'extrait actif selon l'invention soit une quantité représentant 0,65 kg d'huile.

L'huile produite est un liquide homogène et présente une couleur jaune foncée.

Cette huile sera nommée ci-après l'extrait E2 selon l'invention de l'algue Phaeodactylum Tricornutum.

**Exemple 2- Evaluation de l'effet d'un extrait par exemple E2 de l'exemple 1-2 précité, comme agent favorisant l'activité du protéasome des cellules de la peau humaine, en particulier des kératinocytes en l'absence et en présence d'irradiation U.V.**

L'extrait de l'algue Phaeodactylum utilisé dans le présent exemple est l'extrait E2 obtenu par le procédé d'extraction précédemment décrit dans le cadre de l'exemple 1-2.

Les études décrites ci-après ont été réalisées sur des kératinocytes humains normaux (KHN).

L'action de l'extrait E2 selon l'invention sur les cellules de la peau est mesurée de deux manières différentes, soit à partir de 3 activités protéolytiques du protéasome, soit en évaluant la quantité de protéines oxydées qui se sont accumulées de manière plus ou moins importante en fonction de la variation de l'activité protéasomique.

**1)** Principe des tests

**1.1)** Mesure des 3 activités enzymatiques représentatives de l'activité du protéasome

Le premier type de mesures concerne trois activités représentatives du protéasome : la chymotrypsine similaire, l'hydrolase post-glutamique et enfin la trypsine similaire. Elles sont de plus portées par 3 sites catalytiques différents. Chaque activité peptidase du protéasome est déterminée en utilisant un substrat peptidique fluorogène spécifique de chacune des activités, en présence et en l'absence d'un inhibiteur spécifique du protéasome. Le principe du dosage consiste alors à suivre au cours du temps l'augmentation de fluorescence due à la libération des fluorophores issus des peptides fluorogènes grâce à un spectrofluorimètre.

**1.2)** Mesure de la quantité de protéines oxydées

Le second type de mesures est utilisé en complément des mesures d'activités enzymatiques pour évaluer par une méthode classique de détection, la quantité de protéines oxydées. Celle-ci varie en sens inverse de l'activité du protéasome, cette mesure permet ainsi de mesurer l'action de l'agent cosmétique selon l'invention sur l'activité du protéasome.

La technique Oxyblot (western blot) employée pour la détection des protéines oxydées est mise en oeuvre de manière classique comme décrit par Leammli U. K. dans « Cleavage of structural protein during the assembly of the head of the bacteriophage T4 » Nature, 1970, 277, 680-685.

La partie relative à l'immunodétection des protéines sur membrane employée dans cette technique bien connue a été, quant à elle, spécialement adaptée à partir de l'incubation par les anticorps. Elle est décrite au 2.6).
Dans le cas d'une exposition aux UV, des cultures primaires de kératinocytes humains sont irradiés par des UVA et des UVB avec des doses respectivement de 10 joules/$cm^2$ et de 0.05 joules/$cm^2$. Ces valeurs sont constantes pour toutes les expériences.

**1.3)** Mesure de la quantité de protéines (protéasome)

Parallèlement aux mesures d'activités effectuées par Oxyblot, une autre technique de détection de protéines est mise en oeuvre afin de déterminer systématiquement la quantité de protéines présentes dans chaque prise d'essai de chaque échantillon. Cette technique est connue sous le nom de méthode de Bradford. Elle est employée ici de manière classique comme décrit par Bradford, M., dans « A rapid and sensitive method for the quantitation of microgram quantities of protein utilizing the principle of protein-dye binding, » Anal. Biochem., 72,248 (1976). Cette technique est mise en oeuvre systématiquement du fait du protocole expérimental choisi pour les mesures d'activités. En effet, les expériences ci-après décrites sont effectuées à quantité de protéines constante avec des volumes de prise d'essai différents qui sont ensuite complétés à 200 µl (volume total constant).

**2)** Matériel et méthodes

**2.1)** Culture de kératinocytes humains normaux (KHN)

Les cultures de kératinocytes humains normaux (KHN) sont réalisées à partir de prélèvements de peau.
Le prélèvement est, dans un premier temps, rincé 4 fois dans du PBS (Phosphate Buffered Saline-Sigma) (tubes 50 ml). Il est ensuite décontaminé en le trempant dans deux bains successifs d'éthanol à 70% pendant 3 secondes. Lorsque le prélèvement est décontaminé, il est placé dans une boîte de pétri contenant du PBS. On découpe alors des bandelettes de 1 mm de largeur en prenant soin d'éliminer un maximum de tissu adipeux et de derme. Les bandelettes sont placées au fur et à mesure dans une boîte de pétri contenant du PBS. Pour pouvoir récupérer les kératinocytes de l'épiderme, on place les bandelettes pendant 4h à 37°C dans une solution de trypsine à 25% dans du PBS.
On dissocie ensuite le derme de l'épiderme par grattage des bandelettes au scalpel, les cellules épidermiques obtenues sont mises en suspension dans un tube contenant du milieu DMEM (Dulbecco Modifie Eagle Medium-Gibco) + 10% de SVF (Sérum de veau foetal - Eurobio). Après homogénéisation de la suspension, on élimine la partie superficielle constituée de cellules de la couche cornée et on filtre sur un tamis.
La partie filtrée est centrifugée pendant 5 minutes à 176 g. Le culot est repris avec du milieu KHN-D (DMEM + 10% SVF + hydrocortisone 0,4 µg/ml + EGF 10 ng/ml (Epidermal Growth Factor) + toxine cholérique $10^{-9}$M). Les cellules sont comptées puis ensemencées à 15 x $10^6$ cellules/flacon.
Après 24 h de culture, le milieu est changé, les cellules sont rincées avec du PBS et on utilise pour la suite de la culture du milieu de prolifération K-SFM (Gibco).
Les kératinocytes se repiquent de manière tout à fait classique, mais il est nécessaire de les repiquer à 60-70% de la confluence pour qu'ils gardent leurs capacités à proliférer. Ainsi, lorsque les cellules sont à 60-70% de la confluence, le milieu d'entretien est éliminé et le tapis cellulaire est rincé avec du PBS. Les cellules sont mises en présence de 3 ml d'une solution de trypsine-EDTA, puis lorsque les cellules se détachent, la trypsine est inhibée par du milieu avec 10% de SVF (Eurobio). La suspension cellulaire est homogénéisée, récupérée puis centrifugée à 20 g pendant 5 minutes à température ambiante. Le culot ainsi obtenu est repris avec du milieu seul. Pour l'entretien, l'ensemencement se fait comme précédemment à $10^6$ cellules/75 $cm^2$ dans des flacons ventilés et conservés dans les conditions citées plus haut. La confluence est obtenue au bout d'une dizaine de jours et les cellules peuvent être amplifiées pendant 6 à 7 passages.
Dans les cas où l'on souhaite tester l'activité d'une substance sur ces kératinocytes, on effectuera les tests à partir du milieu de culture avec kératinocytes, au moment de la confluence décrite ci-dessus.

**2.2)** Tampon de lyse :

La préparation du tampon de lyse nécessite au préalable la préparation des solutions suivantes:

⇒ Tris-HCL 1,5M, pH=7,5 :

45,375g de Tris base (Sigma ; T1503) à dissoudre dans 200ml d'eau distillée, ajuster le pH à 7,5 avec HCl 12N puis compléter à 250ml

⇒ Solution de saccharose à 1 M (Merck ; réf ;7654)
8,55g de saccharose à dissoudre dans 25 ml d'eau distillée.
⇒ Solution de $MgSO_4$ à 2mM (Sigma ; réf.M7506)
0,01g de $MgSO_4$ à dissoudre dans 20ml d'eau distillée
⇒ Solution de Triton X100 à 4% (Sigma ; réf.X100)
0,8g de Triton X 100 à dissoudre dans 20ml d'eau distillée, puis aliquoter à 0,5ml et conserver à -20°C
⇒ Solution de PMSF à 40mM (Sigma ; réfP7626)
14mg de PMSF à dissoudre dans 2ml d'éthanol absolu, aliquoter à 50µl et conserver à -20°C
⇒ Solution de leupeptine à 0,5mg/ml (Sigma ; réf L2884 ; conservé à - 20°C).
Aliquoter à 50µl et conserver à -20°C
⇒ Solution de DL-Dithiothréitol à 1 M (Sigma ; réf.D0632 conservé à 4°C).
0,154g de DL-dithiothréitol à dissoudre dans 1 ml d'eau distillée. Aliquoter à 10µl et conserver à -20°C.

En vue de la préparation de 100ml de tampon de lyse, il faut tout d'abord réaliser la solution dite incomplète décrite dans le tableau 1 et aliquoter à 4,39ml (c'est à dire diviser en petits volumes tous égaux à 4,39ml) et conserver à -20°C :

**Tableau 1**

| Solution incomplète pour préparer le tampon de lyse | | |
|---|---|---|
| Solutions | Volumes | Concentrations finales |
| Tris-HCl 1,5 M pH= 7,5 | 0,33 ml | 5 mM |
| Saccharose 1 M | 25 ml | 2,25 M |
| $MgSO_4$ 2 mM | 10 ml | 0,2 mM |
| EDTA 500 mM | 4 ml | 20 mM |
| Eau distillée | 48,47 ml | |

Le tampon de lyse ou solution dite complète est préparé extemporanément avec 4,39ml de solution incomplète + 500µl de Triton X-100 à 4% + 10µl de DTT 1M + 50µl de leupeptine 0,5mg/ml + 50 µl de PMSF à 40mM.

**2.3)** Dosage des protéines par la Méthode de Bradford

La mise en oeuvre est classique et nécessite la préparation de la gamme étalon suivante :

A partir d'une solution mère de BSA : 50µg/ml (BIORAD ; protéine standard ; réf.500-0006).

Dans chaque tube : on additionne 200µl de bleu de Coomassie G250.

Ledit bleu est préparé extemporanément par dilution au 1/5 de la solution mère.

Pour la préparation des échantillons, on récupère des cellules dans le tampon de lyse puis on les sonique avant de doser leur concentration en protéine.

Si la concentration en protéine des échantillons est supérieure à 3mg/ml, il faut les diluer au 1/100, puis prendre 100µl d'extrait cellulaire dilué par 700µl d'eau et 200µl de bleu. Si la concentration est faible, il faut alors prendre 10µl d'extrait cellulaire avec 790µl d'eau et 200µl de bleu de Coomassie. Vortexer. Après 5 minutes d'attente, on effectue une lecture des résultats à 595nm avec un spectrofluorimètre FluoStar BMG Lesdits résultats sont reportés dans le tableau 2.

TABLEAU 2

| Dosage des protéines (méthode de Bradford) : Etalonnage | | |
|---|---|---|
| Qté Prot. (µg/tube) | BSA (µl) | H2O (µl) |
| 0 | 0 | 800 |
| 1 | 20 | 780 |
| 2 | 40 | 760 |

(suite)

| Dosage des protéines (méthode de Bradford) : Etalonnage | | |
|---|---|---|
| Qté Prot. (μg/tube) | BSA (μl) | H2O (μl) |
| 3 | 60 | 740 |
| 4 | 80 | 720 |
| 5 | 100 | 700 |
| 6 | 120 | 680 |
| 8 | 160 | 640 |

**2.4)** Préparation des échantillons en vue des essais

**2.4.1)** Préparation des échantillons en vue d'un dosage d'activité

Le principe général de culture, du traitement à base de l'extrait E2 et de l'irradiation par les UVA et UVB est décrit au schéma 1, en fin de description. Les KHN en culture selon 2.1) sont récupérés et lysés grâce à 2.2) à des temps différents selon l'expérience envisagée, c'est à dire avant ou après traitement par l'extrait selon l'invention et/ou avant ou après irradiation par les UV. Les protéasomes recherchés en vue d'être analysés sont présents dans le surnageant de ces lysats cellulaires.

Les différents types d'échantillons sont les suivants :

- I1 = témoin + extrait E2 seul
- I2 = témoin + extrait E2 avant U.V.
- I3 = témoin + extrait E2 après U.V.

Chaque volume de surnageant est divisé en trois fractions aliquotes à chaque fois et les fractions d'un même échantillon sont placées sur un lecteur microplaque. Les substrats peptidiques fluorogènes spécifiques de chaque activité sont rajoutés (en fonction de la quantité de protéines). Les manipulations au spectrofluorimètre décrites ci-après sont effectuées avec un volume total constant mais avec des prises d'essais de volumes Vi différents pour chaque échantillon. Le fait de travailler en quantité de protéines constante pour des Vi différents implique de contrôler au préalable par la méthode de Bradford décrite au 2.3) la quantité de protéines introduites.

On cherche ensuite à rapporter une quantité de protéines présente par mg de protéines introduites.

**2.4.2)** Préparation des échantillons en vue d'un dosage pour la mesure des quantités de protéines oxydées

On applique le même principe général de culture décrit au schéma 1. La technique de l'Oxyblot (Western blot) ou électrophorèse de protéines fait migrer les échantillons Ii sur le gel. Ils sont ensuite transférés sur une membrane qui est incubée avec l'anticorps désiré. Pour la mesure de la quantité de protéines oxydées, on effectue une incubation avec un anticorps polyclonal anti-dinitrophényl (anti-DNP) pour détecter les groupements carbonyles des protéines ayant réagi avant d'effectuer le test d'activité.

**2.5)** Dosage des activités enzymatiques du protéasome

a) Préambule

Pour le dosage des activités enzymatiques du protéasome, les KHN cultivés sont rincés deux fois avec du PBS puis chaque activité peptidase du protéasome est déterminée en utilisant un substrat peptidique fluorogène spécifique de chacune des activités, en présence et en absence d'un inhibiteur spécifique du protéasome, le MG 132 (N-Cbz-Leu-Leu-Leucinal). Les substrats peptidiques sont les suivants: Leu-Leu-Val-Tyr-aminométhylcoumarine (LLVY-amc) pour l'activité chymotrypsine similaire, Leu-Leu-Glu-β-naphthylamine (LLE-na) pour l'activité hydrolase post-glutamique et Leu-Ser-Thr-Arg-amc (LSTR-amc) pour l'activité trypsine similaire. Le principe du dosage consiste à suivre au cours du temps l'augmentation de fluorescence due à la libération des fluorophores aminométhylcoumarine (amc) ou β-naphthylamine (na) issus des peptides fluorogènes grâce à un spectrofluorimètre.

Pour mesurer l'effet des U.V. sur le protéasome, des kératinocytes humains normaux ou KHN en culture sont irradiés par des doses constantes d'UVA à 10J / $cm^2$ et d 'UVB à 0.05J / $cm^2$, puis ils sont récupérés et lysés à des

temps différents après l'irradiation.

b) Les trois activités protéolytiques du protéasome

Les activités chymotrypsine similaire, Hydrolase post-glutamique et Trypsine similaire sont mesurées à partir de protéasomes extraits des lysats cellulaires en utilisant les peptides fluorogènes comme substrat aux concentrations suivantes : LLVY-amc à 12.5 μM, LLE-na à 150 μM et LSTR-amc à 40 μM. Pour chaque série de mesures, il est nécessaire de refaire une incubation avec le substrat fluorescent spécifique de chaque activité. Ces activités sont mesurées en parallèle avec un inhibiteur spécifique du protéasome : le MG132 (N-Cbz-Leu-Leu-leucinal) à 20 μM, afin d'évaluer la part d'activité indépendante du protéasome.

**2.5.1)** Mode opératoire pour la détermination de l'activité LLVY (Chymotrypsine similaire)

Son principe, qui est le suivant, est d'ailleurs identique aux réactifs près pour les trois activités.
L'activité enzymatique dite « chymotrypsine similaire » est l'activité similaire à la chymotrypsine,qui se traduit par une coupure après un acide aminé aromatique, ici la tyrosine.

**Protéasome (activité LLVY)**

**N-Succinyl-LLVY-amc ⟶ N-Succinyl- LLVY + amc fluorescent**

Par réaction de clivage, le pigment fluorogène est liberé. Ainsi, une lecture au spectrofluorimêtre (FluoStar (BMG)) permet d'accéder dans un premier temps aux résultats bruts exprimés en unité de fluorescence par minute (U.F/min). Cette unité est arbitraire car elle est liée à l'appareil utilisé.
Nous avons arbitrairement choisi d'exprimer l'activité du protéasome en pmol de amc libéré par min et par mg de protéines (protéasome) présentes dans l'échantillon. Pour cela, on dose au préalable dans chaque échantillon issu des cultures de KHN la quantité de protéines (protéasome) par la méthode de Bradford. Les expériences sont effectuées avec des volumes Vi différents, seul le volume réactionnel total reste constant et égal à 200μl. On réalise également au préalable une courbe d'étalonnage en amc afin de pouvoir corréler les résultats fournis par l'appareil sur un échantillon donné li et ceux pour lesquels la quantité de protéines est connue.
On utilise pour cela les réactifs suivants :

- Tampon TRIS 25mM pH 7,5
- Pour la courbe d'étalonnage : 7- amino- 4-méthylcoumarine (amc) (Sigma : A9891)
  Solution stock à 20mM (3,5mg /1ml DMSO)
- Pour la mesure d'activité : Substrat fluorogénique : N-Succinyl-Leu-Leu-Val-Tyr-7-amino-4-Méthyl coumarine (Sigma : S6510) Solution stock à 10mM dans le DMSO

On réalise une gamme d'étalonnage en amc. Pour cela on dilue la solution stock de amc à 4μM dans le tampon TRIS. Dans une plaque de 96 puits, on distribue chaque quantité en double.
Faire un blanc avec 200μl de tampon TRIS. Puis lire les résultats au spectrofluorimètre à une longueur d'onde d'excitation de 350nm et une longueur d'onde d'émission de 440nm
Les résultats obtenus pour la courbe d'étalonnage (droite de pente a) sont rapportés au tableau 3 ci-après :

TABLEAU 3

| COURBE D'ETALONNAGE en AMC | | |
|---|---|---|
| Quantité de AMC. (μM) | AMC (μl) | TRIS (μl) |
| 0 | 0 | 200 |
| 0,1 | 5 | 195 |
| 0,2 | 10 | 190 |
| 0,3 | 15 | 185 |
| 0,4 | 20 | 180 |

(suite)

| COURBE D'ETALONNAGE en AMC | | |
|---|---|---|
| Quantité de AMC. (μM) | AMC (μl) | TRIS (μl) |
| 0,5 | 25 | 175 |
| 0,6 | 30 | 170 |
| 0,7 | 35 | 165 |
| 0,8 | 40 | 160 |
| 1,0 | 50 | 150 |

Pour le dosage de l'activité LLVY, on introduit en double dans une plaque de 96 puits un volume fixe de lysat cellulaire (déterminé par la méthode de Bradford afin d'introduire à terme la même quantité de protéines à chaque fois). En pratique, c'est la concentration la plus faible en protéines des échantillons (qui doit correspondre à 20μg de protéines). On complète à 100μl avec du tampon TRIS.

On réalise ensuite une incubation des échantillons en ajoutant 100μl du substrat peptidique fluorogène de l'activité LLVY en le diluant préalablement à 25μM dans le tampon TRIS (pour une concentration finale de 12,5μM).

Puis la lecture des résultats est effectuée à l'aide du spectrofluorimètre à une longueur d'onde d'excitation de 355nm et une longueur d'onde d'émission de 460nm au gain 40 toutes les 2 minutes et ceci pendant 30 minutes.

Les résultats bruts sont exprimés en U.F./min, unité propre à l'appareil.

Le dosage est réalisé sur un volume réactionnel constant de 200μl contenant un volume Vi de lysat cellulaire fixé en fonction des concentrations de protéines (protéasome) des lysats.

La concentration des protéines dosées extemporanément est exprimée en μg/μl.

A partir de la gamme étalon, l'activité peut être exprimée en pmol d'amc libéré/minute/mg de protéines grâce à la formule empirique suivante :

$$\frac{\text{Vitesse moy U.F/min (valeur fournie par l'appareil)} \times 200 \times 10^{-6} \times 10^{-6} \times 10^{12}}{\text{Protéine } \mu g/\mu l \text{ (mesurée par Bradford)} \times Vi.10^{-3} \times \text{coefficient de la pente a } (a=4{,}568.10^{4})}$$

Pour chaque échantillon li aliquoté, on cherche à déterminer la quantité de amc libéré et à l'exprimer en pmol/mn/mg de protéine. Pour cela, on utilise la courbe d'étalonnage. Le quotient entre la grandeur lue au spectrofluorimètre et la pente de ladite courbe nous donne un premier résultat intermédiaire exprimé en μmol/l/min de protéines. Il suffit ensuite de multiplier cette valeur par le volume réactionnel final (200μl) et de diviser par le volume Vi de la prise d'essai de l'échantillon li à doser ainsi que par la quantité de protéines (μg) dosée extemporanément par la méthode de Bradford. Le résultat final peut alors être exprimé, aux coefficients multiplicateurs près, en pmol/min/mg de protéines.

Ri = (N .Vt/ Vi.Qi).coefficient
N= Nbre de mole de protéines libérées par litre et par minute
Vt = Volume réactionnel final (200μl)
Vi = Volume d'une prise d'essai pour l'échantillon li (μl)
Qi = Quantité de protéines (μg) dosée par la méthode de Bradford

Si l'on applique la formule décrite ci-dessus, Ri représente la quantité de protéines (protéasome) recherchées exprimée arbitrairement en pmol/mn/mg de protéines (protéasome) introduites.

**2.5.2)** Mode opératoire pour la détermination de l'activité LSTR (activité trypsine similaire)

On étudie la réaction de clivage suivante :

**Protéasome(activité LSTR)**

**Nt Boc LSTR-amc → Nt Boc LSTR + amc fluorescent**

L'activité LSTR est accessible par une formule identique en réalisant au préalable un étalonnage en amc à partir des réactifs suivants :

-Tampon TRIS 25mM pH 7,5
-Pour la courbe d'étalonnage : 7-amino-4-méthylcoumarine (amc) (Sigma : A9891)
Solution stock à 20mM (3,5mg /1ml DMSO)

La lecture des résultats bruts est effectuée grâce au spectrofluorimètre réglé à une longueur d'onde d'excitation de 350nm et une longueur d'onde d'émission de 440nm.

-Pour les mesures d'activité : Substrat fluorogénique : N-t-BOC-Leu-Ser-Thr-Arg-7-amino-4-méthylcoumarine (Sigma : B4636)
Solution stock à 10mM dans le DMSO
inhibiteur du protéasome: Mg132 (Z- Leu- Leu-Leu- CHO) (Affinity, ZW8440)
Solution stock à 20mM dans le DMSO

Les résultats obtenus pour la courbe d'étalonnage (droite de pente b) sont rapportés au tableau 4 ci-après

TABLEAU 4

| Courbe d'étalonnage en amc | | |
|---|---|---|
| Qté de AMC (μM) | AMC (μl) | TRIS (μl) |
| 0 | 0 | 200 |
| 0,1 | 5 | 195 |
| 0,2 | 10 | 190 |
| 0,3 | 15 | 185 |
| 0,4 | 20 | 180 |
| 0,5 | 25 | 175 |
| 0,6 | 30 | 170 |
| 0,7 | 35 | 165 |
| 0,8 | 40 | 160 |
| 1,0 | 50 | 150 |

Pour les mesures d'activité, la lecture des résultats est faite avec le Fluostar (BMG) à 355nm (longueur d'onde d'excitation) et 460nm (longueur d'onde d'émission) au gain 30 toutes les 2 min et ceci pendant 30 min.

On peut également accéder aux résultats par une autre formule empirique similaire à la précédente:

$$\frac{\text{Vitesse moy U.F. / min (valeur fournie par l'appareil)} \times 200 \times 10^{-6} \times 10^{-6} \times 10^{12}}{\text{Protéine } \mu g/\mu l \text{ (mesurée par Bradford)} \times Vi.10^{-3} \times \text{coefficient de la pente b } (b= 1{,}728.10^{4})}$$

**2.5.3)** Mode opératoire pour la détermination de l'activité LLE (activité hydrolase post-glutamique)

On étudie la réaction de clivage suivante :

$$\text{N-CBZ-LLE-na} \xrightarrow{\text{Protéasome(activité LLE)}} \text{N-CBZ- LLE + na fluorescent}$$

On réalise sur le même schéma un étalonnage cette fois par rapport au substrat β-naphtylamine (na) à partir des réactifs suivants :

- Tampon TRIS 25m M, pH =7,5
- Pour la courbe d'étalonnage : β-Naphtylamine (na) (Sigma : N8381) Solution stock à 20mM (5,73mg /2ml DMSO)

Les résultats sont recueillis grâce au spectrofluorimètre à une longueur d'onde d'excitation de 333nm et une longueur d'onde d'émission de 410nm.

Les résultats obtenus pour la courbe d'étalonnage (droite de pente c) en na sont rapportés au tableau 5 ci-après :

Tableau 5

| Courbe d'étalonnage en na | | |
|---|---|---|
| Qté de na (µM) | na (µl) | TRIS (µl) |
| 0 | 0 | 200 |
| 0,1 | 5 | 195 |
| 0,2 | 10 | 190 |
| 0,3 | 15 | 185 |
| 0,4 | 20 | 180 |
| 0,5 | 25 | 175 |
| 0,6 | 30 | 170 |
| 0,7 | 35 | 165 |
| 0,8 | 40 | 160 |
| 1,0 | 50 | 150 |

- Pour les mesures d'activité : Substrat fluorogénique N - CBZ- Leu-Leu-Glu-ß -Naphtylamine (Sigma : C0788) Solution stock à 10mM dans le DMSO.

Pour la lecture des mesures, on utilise un appareil FLUOstar (BMG) à 340nm (longueur d'onde d'émission)et 410nm (longueur d'onde d'excitation) au gain 83 toutes les 2 minutes pendant 35 minutes.

En utilisant la même démarche que pour les activités précédentes, une mesure de l'activité LLE est alors accessible par la formule empirique suivante dans laquelle la quantité de protéine (protéasome) est exprimée en pmol de na libérée/minute/mg de protéines (protéasome) introduites :

$$\frac{\text{Vitesse moy U.F. / min (valeur fournie par l'appareil)} \times 200 \times 10^{-6} \times 10^{-6} \times 10^{12}}{\text{Protéine µg/µl (mesurée par Bradford)} \times \text{Vi}.10^{-3} \times \text{coefficient de la pente c } (c= 0{,}966 .10^{4})}$$

**2.6)** Détection de la quantité de protéines oxydées accumulées:

**2.6.1)** détection par OXYBLOT (Western Blot)

Il s'agit de mettre en évidence les groupements carbonyles (marqueurs de l'oxydation) qui ont été introduits dans les chaînes protéiques par l'intermédiaire des espèces sensibles à l'action de l'oxygène (ROS pour Reactive Oxygen Species) ou bien à d'autres mécanismes d'oxydation tels que la glycation/glycoxydation ou bien la lipoperoxydation, selon un mécanisme site-spécifique.

Le principe est le suivant. Les groupes carbonyles dans les chaînes réagissent avec du 2,4-dinitrophenylhydrazine (DNPH) pour donner un dérivé hydrazone. Les échantillons marqués au DNP sont séparés sur un gel de polyacrylamide par électrophorèse suivi d'un transfert sur une membrane de nitrocellulose comme pour un Western Blot classique. Ensuite la membrane est incubée en présence du premier anticorps spécifique de la molécule de DNP liée aux protéines possédant un groupement carbonyle. L'étape suivante est l'incubation avec l'anticorps secondaire (anti-lapin) qui est couplé à la peroxydase. La révélation se fait à l'aide des réactifs décrits au 2.6.2)).

**2.6.2** : Protocole

Tous les réactifs utilisés proviennent du kit OXYBLOT (Appligene-Oncor, Illkirch, France).

A- Préparation des échantillons et électrophorèse

Nous utilisons une quantité de protéines comprise entre 15-20 μg provenant de 5 μl de lysats de kératinocytes, irradiés ou non. 10 μl de 2,4 dinitrophénylhydrazine sont ajoutés ainsi que 5 μl de SDS à 12 %, puis on laisse agir 15 min à température ambiante. Ensuite 7,5 μl de solution neutralisante (solution fournie dans le kit) sont ajoutés. Les échantillons sont alors prêts à être déposés.

L'électrophorèse des protéines à faible poids moléculaire est réalisée sur gel de polyacrylamide de 1 mm d'épaisseur à une concentration de 12,5%, en conditions dénaturantes et réductrices, en tampon discontinu selon la méthode de Laemmli (1970). Les gels à 12,5% de T (T=acrylamide+bis-acrylamide), 2,7% de C (C=(bis-acrylamide/acrylamide) + bis-acrylamide) permettent de séparer les protéines d'une masse moléculaire variant de 20 à 120 kDa. L'appareil employé provient de la société Hoefer. Toutes les solutions nécessaires à l'élaboration des gels sont présentées ci-dessous :

I) Tampons et solutions utilisés pour la préparation des gels d'électrophorèse en conditions dénaturantes et réductrices, en tampon discontinu

Solution de monomères :

40% acrylamide / Bis-acrylamide, 2,6% C (Biorad ; réf.161- 0148)

Tampon de gel de résolution: Tris-HCl 1,5 M, pH=8,8
Dissoudre 18,15g de Tris base (Sigma; T1503) dans 100ml d'eau distillée, et ajuster le pH à 8,8 avec de l'HCl 12N

Tampon de gel de concentration : Tris-HCl 0,5M, ph=6,8
Dissoudre 6g de Tris base dans 100ml d'eau distillée, et ajuster le pH à 6,8 avec de l'HCl 12N

Tampon de migration 10X : Tris-HCl 0,25 M, pH=8,3, glycine 1,92M; SDS 1%
On utilise les réactifs suivants : 12g Tris base, 57,6g de glycine (Research Organics Inc ; 5037G) ; 40ml de SDS à 10% (Sigma ; L5750) et compléter à 400ml avec de l'eau distillée
Ces solutions sont conservées à 4°C

Persulfate d'ammonium $(NH_4)_2S_2O_8$ : (Sigma; A1433 à 10% soit 100mg/ml. La solution est aliquotée et conservée à -20°C.

Tampon d'échantillon réducteur 4X : 2,5ml de Tris-HCl 0,25M, pH=6,8 ; 0,4g de SDS 8% ; 2ml de Glycérol à 40% ; 1ml de β-mercaptoéthanol à 20% ; une pointe de spatule de Bleu de Bromophénol à 0,02%. Cette solution se conserve à température ambiante.

Standards précolorés à bas poids moléculaires (Biorad ; réf.161-0305). Ils sont composés de Phosphorylase B

(104 kda), Bovine serum albumin (82 kda), Ovalbumin (48,3 kda), Carbonic anhydrase (33,4 kda), Soybean trypsin inhibitor (28,3 kda), Lysozyme (19,4 kda).

II) Gels d'électrophorèse

Préparation du gel de résolution à 12,5% T

Tableau 6

| Solutions | Volumes pour un gel (10ml) | Concentration finale |
|---|---|---|
| Solution de monomère | 3,12 ml | T; 2,7% C |
| Tampon de gel de résolution | 2,5 ml | 0,375 M |
| SDS 10% | 100 µl | 0,1% |
| Persulfate d'ammonium (10%) | 70 µl | 0,07 % |
| TEMED (Research Organics Inc; 3009T) | 10 µl | |
| Eau distillée | 4,2 ml | |

Préparation du gel de concentration à 4% T

Tableau 7

| Solutions | Volumes pour deux gels (10ml) | Concentration finale |
|---|---|---|
| Solution de monomère | 1 ml | T; 2,7% C |
| Tampon de gel de résolution | 2,5 ml | 0,175 M |
| SDS 10% | 100 µl | 0,1% |
| Persulfate d'ammonium (10%) | 70 µl | 0,07 % |
| TEMED (Research Organics Inc, 3009T) | 10 µl | |
| Eau distillée | 4,2 ml | |

Préparation du gel de séparation

Ce gel peut être coulé soit la veille, soit le jour même, mais de toute façon deux heures avant la migration.

Le moule utilisé permet de couler deux gels. Le montage est réalisé comme suit: on dispose dans l'ordre les joints rouges, les joints noirs graissés, un plastique, une grosse plaque, un plastique, une plaque alumine, les spacers (blancs pour les gels de 1 mm; noirs pour les gels de 1,5 mm), une plaque de verre, un plastique, une plaque alumine, les spacers, une plaque de verre, trois plastiques puis le couvercle. Le montage ainsi obtenu est maintenu par des pinces et placé sur une zone plane.

Le coulage du gel est réalisé à l'aide d'une pipette P5000 jusqu'à environ 0,5 mm du bas du peigne prévu pour le gel de concentration. Le peigne est enlevé puis de l'eau est ajoutée très doucement pour qu'ils soient plans (+ ou - 0,5 ml d'eau/gel).

Le montage qui est recouvert d'aluminium ne doit pas être bougé pendant la durée de la polymérisation.

Préparation du gel de concentration (Stacking Gel)

Les gels de séparation (entre les plaques d'alumine et de verre) sont retirés du moule et placés sur l'appareil d'électrophorèse. S'il n'y a qu'un seul gel, celui-ci doit être remplacé par une plaque de verre.

Le peigne est inséré entre la plaque d'alumine et celle de verre. Le gel est ensuite coulé avec une pipette Pasteur de transfert en polyéthylène (Biorad, réf.223-9528). Avant la polymérisation, le niveau du gel est vérifié et ajusté

si le volume échantillon est élevé. Après une heure, le gel est polymérisé.

Préparation des échantillons

Avant de récupérer les cellules contenues dans les boîtes, elles sont rincées au PBS, 2 fois. Après le dernier rinçage, le PBS est éliminé au maximum. Les cellules sont récupérées dans du tampon de lyse (voir Schéma 1) par grattage ($5.10^6$ cellules/ml de tampon de lyse minimum): Les lysats sont congelés à -80°c.
Avant de déposer, les lysats décongelés sont soniqués puis les protéines dudit échantillon sont dosées.
Le volume à déposer est fonction de la quantité de protéines (quantité maximale de protéines : 60 μg ; vol. maxi = 25μl, vol.mini = 5μl). Suite aux essais préliminaires, le volume de chaque échantillon déposé correspond à 10 μg de protéines.
Le dosage des protéines a été réalisé pendant la polymérisation du gel de séparation.

Dépôts

250 ml de tampon de migration sont versés sur le gel, entre le gel, la plaque de verre et l'appareil d'électrophorèse ainsi que dans la cuve.
Les échantillons sont alors centrifugés à 10000 G pendant 5 mn puis déposés. 10μl de témoins précolorés (Biorad, Prestained SDS-PAGE standards-réf. 161-0305) sont aussi déposés ainsi que 0,3μg de HSP32 (TEBU, réf. SPP-730 aliquotée à 0,1 μg/ml et congelée à -20°c).

Migration

L'électrophorèse est réalisée à température ambiante, sous réfrigération, avec un voltage non limitant de 300V et avec un ampérage constant (15 mA) pendant la migration dans le gel de concentration. L'ampérage est diminué à 10 mA une fois que les échantillons ont atteint le gel de séparation.
L'électrophorèse est arrêtée lorsque le front de migration atteint le bas du gel (environ 1 heure 30 de migration).

B- Transfert des protéines sur membrane et blocage des sites de liaisons spécifiques

Une fois la migration terminée, le gel est équilibré pendant 20 minutes à température ambiante, sous agitation, dans le tampon de transfert à l'origine de cette méthode décrit par Towbin H., Staehelin T. and Gordon J. dans « Electrophoretic transfer of proteins from polyacrylamide gels to nitrocellulose sheets : procedure and some applications » Proc. Natl. Sci., USA, 1979, 76, 4350-4354.
Une membrane de difluorure de polyvinylidène (PVDF) (Biorad, réf. 162-0184), de bonne tenue mécanique et de forte capacité de fixation des protéines, est trempée dans du méthanol à 100% jusqu'à transparence puis équilibrée dans le tampon de transfert jusqu'à son immersion complète (20 min).
Dans l'appareil de transfert semi-sec (Biorad), on introduit une feuille de papier filtre épaisse (Biorad, réf. 17033960) préalablement trempée dans le tampon de transfert, la membrane de PVDF, le gel, puis à nouveau une feuille de papier filtre mouillée est déposée sur l'anode il faut prendre garde à bien éliminer toutes bulles d'air entre les différentes couches à l'aide d'une tige en verre afin d'éviter tout risque de transfert. L'appareil est ensuite fermé par un couvercle jouant le rôle de la cathode.
Le transfert des protéines est réalisé à ampérage non limitant (5,5 mA :$cm^2$, 250 mA) et à voltage constant (15V) pendant 30 minutes.
La membrane est alors placée sous agitation pendant une nuit à 4°C dans une solution de blocage des sites de liaisons spécifiques constituée de 10% (25g) de lait écrémé (Régilait) dans du tampon TBS-T (25ml).

C-Réaction antigène-anticorps : Immunodétection des protéines sur membrane de PVDF

On utilise le kit ECL (Enhanced ChemiLuminescence) pour révéler la peroxydase à l'aide de son substrat
Après blocage des sites non spécifiques, la membrane est rincée une fois pendant 15 minutes et deux fois pendant 5 minutes dans du TBS-T.
Ensuite, cette membrane est mise en présence de l'anticorps primaire de lapin Anti-DNP dilué au 1/150 pendant une heure sous agitation à température ambiante.
Elle est ensuite rincée une fois 15 minutes et deux fois 5 minutes dans du TBS-T afin d'éliminer l'excès d'anticorps libre non fixé.
Puis elle est mise en contact avec l'anticorps secondaire anti-lapin couplé à la peroxydase (Amersham ; réf. NA934 ; conservé à 4°C) dilué au 1/5000 dans le TBS-T (5ml) sous agitation à température ambiante.

Après une heure d'incubation, elle est rincée rapidement deux fois avec du tampon TBS-T, puis lavée une fois pendant 15 minutes et enfin quatre fois pendant 5 minutes avec le tampon TBS-T.

Après avoir été égouttée, elle est placée sur un film alimentaire (SARAN), du côté protéine, c'est à dire vers le haut.

La membrane est révélée à l'aide d'un kit de détection hautement sensible par chimioluminescence (Amersham ; ECL Western blotting réf. RPN2106), utilisant le luminol comme substrat de la peroxydase. Sous l'action de la peroxydase et d'un amplificateur, le luminol est oxydé et passe dans un état transitoire excité. Le retour à l'état fondamental se fait par émission de photons qui vont ensuite interagir avec un film autoradiographique placé sur la membrane.

1 ml de chacune des deux solutions du kit de détection sont mélangés (2 ml, volume minimum nécessaire au recouvrement de la membrane).

Aussitôt, le mélange est versé uniformément sur la membrane et laissé en contact pendant exactement une minute à température ambiante.

La membrane égouttée est protégée sous un film alimentaire Saran et mise sous cassette à l'abri de la lumière, puis recouverte d'un film autoradiographique pré-flashé (Amersham, Hyperfilm ECL réf. RPN2103).

Après une heure d'exposition, le film autoradiographique est récupéré et plongé 5 minutes dans un révélateur de film (révélateur radio, LX 24 Kodak), puis rincé à l'eau et enfin fixé par immersion durant un minimum de 5 minutes dans le fixateur (fixateur radio, AL 4 Kodak) avant d'être à nouveau rincé à l'eau.

Le film est séché puis les bandes recueillies sont quantifiées grâce au logiciel Gels Analysts 3.01.

3) RESULTATS DES ESSAIS

3.1) Effets de l'extrait E2 sur les activités du protéasome de KHN

Les échantillons utilisés ici ont été préparés conformément au protocole décrit au Schéma 1 pour le témoin et pour l'échantillon traité par l'extrait. Une prise d'essai de 15μl est effectuée et des mesures d'activités sont réalisées conformément à 2.5).

Les résultats présentés dans les tableaux 8, 9, 10 montrent que 7 heures après l'addition de l'extrait de l'algue (Ph), les 3 activités peptidases du protéasome augmentent et ceci de manière significative pour 2 d'entre elles (les activités chymotrypsine similaire et trypsine similaire). Par ailleurs, on observe que dans les KHN traités par Ph pendant 7 heures la quantité de protéines oxydées intracellulaires est plus faible que celle des cellules témoins (figure 4, pistes 1 et 2). Ceci montre que l'effet stimulateur observé de l'extrait selon l'invention sur les activités peptidases du protéasome se traduit par une diminution du taux de protéines oxydées intracellulaires. En pratique, ce test a été réalisé sur une prise d'essai de 8μg de lysat cellulaire. Les 3 types d'échantillons I1, 12 et 13 ont été préparés 7 heures après l'irradiation et puis incubés en présence de DNPH pendant 15 min. La réaction a été ensuite stoppée par une solution neutralisante fournie dans le kit Amersham.

3.2) Effets des UV A+B sur le protéasome de KHN en culture primaire

Des cultures primaires de kératinocytes humains selon 2.1) ont été irradiées par les UVA et les UVB conformément à un protocole répondant au principe du schéma 1. La dose de rayonnement ultraviolet retenue pour cette étude, est de 10 joules/$cm^2$ pour les UVA et 0.05 joule/$cm^2$ pour les UVB. Suite à une irradiation combinée de ces deux doses d'UVA et d'UVB, les trois activités peptidases du protéasome sont mesurées à des temps différents. Pour ce dosage, les cellules sont lysées 1 heure, 3 heures, 7 heures et 24 heures après l'irradiation et la concentration en protéines (protéasome) est déterminée conformément à 2.3). Les mesures d'activités sont effectuées selon 2.5) avec des concentrations de substrat fluorogène égales à 12,5 μM pour LLVY-amc, 150 μM pour LLE-na et enfin 40 μM pour LSTR-amc. Ces activités ont été mesurées en parallèle avec le MG132 (20 μM), afin d'évaluer la part d'activité indépendante du protéasome. Les résultats présentés sur les figures 1, 2 et 3 sont exprimés en % par rapport au témoin non irradié et ils montrent que les trois activités peptidases sont diminuées pour les quatre temps analysés après l'exposition aux UVA+B. Cette expérience démontre que le protéasome voit son activité affectée suite à une irradiation par un rayonnement ultraviolet. On constate également que l'effet est d'autant plus important que le temps qui sépare l'irradiation de la mesure d'activité est plus long (7 h et 24 h).

3.3) Effets d'un extrait d'algue Ph sur les activités du protéasome de KHN lorsqu'il est administré avant irradiation UV A+B :

Il a été nécessaire de rajouter des vitamines anti-oxydantes (50 μM tocophérol et 1 mM d'ascorbyl phosphate) pour préserver l'efficacité de l'extrait E2 pendant et après l'irradiation. Des cultures primaires de kératinocytes humains selon 2.1) ont été traitées par l'extrait selon l'invention (dose de 5μg/ml + vitamines) pendant 48 heures puis irradiées

par les UVA+B et enfin lysées. Les résultats contenus dans les tableaux 11, 12, 13 ci-après montrent que la baisse des activités peptidases du protéasome suite à l'irradiation par les UVA+B est complètement bloquée en présence de l'extrait dans lequel des vitamines ont été ajoutées. Le niveau des activités peptidases du protéasome étant le même pour les KHN témoins non traités/non irradiés que pour les KHN traités/irradiés 24 h après, l'agent cosmétique à base de l'extrait selon l'invention a permis de préserver les activités peptidases du protéasome. Il a donc un effet protecteur vis-à-vis des UVA+B.

3.4-Effets d'un extrait d'algue Phaeodactylum (Ph) sur les activités du protéasome de KHN après irradiation.

Des cultures primaires de kératinocytes humains sont irradiées par la dose d'UVA et d'UVB puis traitées par une quantité de 2,5 µg/ml d'extrait E2 selon l'invention pendant 7 heures. On constate que la quantité de protéines oxydées intracellulaires est augmentée par l'irradiation UV mais dans une moindre mesure quand les cellules ont été traitées par l'extrait E2 selon l'invention. L'expérience est décrite au 3.1) et les résultats sont présentés à la figure 4 (pistes 3 et 4). L'utilisation dudit extrait permet une meilleure élimination des protéines oxydées. D'autre part, les résultats donnés dans les tableaux 14, 15, 16 montrent que le traitement à base de l'extrait E2 restaure les 3 activités du protéasome de manière significative quand le traitement est effectué après l'irradiation. Cette restauration complète des activités du protéasome indique que l'agent à base de Ph a un effet réparateur sur les effets des UVA+B au niveau de l'activité protéasomique.

Comme nous l'avions vu au 3-1), pour les KHN traités par l'extrait E2 selon l'invention, la quantité de protéines oxydées intracellulaires est plus faible que dans l'échantillon témoin (voir figure 13, pistes 1 et 2). Ceci montre que l'effet stimulateur observé par l'utilisation de l'extrait E2 sur les activités peptidases du protéasome se traduit par une diminution du taux de protéines oxydées intracellulaires. Les pistes 3 et 4 de la figure 4 montrent quant à elles que l'irradiation par les UV génère bien une quantité (proportionnelle à l'intensité des tâches) plus importante de protéines oxydées mais celle-ci est nettement moins importante suite à l'utilisation dudit extrait.

**Exemple 1 : Crème de jour pour le visage, anti-âge**

| | |
|---|---|
| Glycéryl stéarate + PEG-100 stéarate | 6,00 % |
| Squalane | 3,00 % |
| Polyisobutène hydrogéné | 3,00 % |
| Tricaprylate/caprate de glycérol | 3,00 % |
| Glycérine | 2,00 % |
| Méthoxycinnamate d'octyl | 2,00 % |
| Cire d'abeille | 1,50 % |
| Octanoate de cétostéaryl | 1,50 % |
| Alcool cétylique | 1,00 % |
| Alcool stéarylique | 1,00 % |
| Diméthicone | 1,00 % |
| Extrait E2 d'algue Phaeodactylum tricornutum, de l'exemple 1.2 | 1,00 % |
| Gomme Xanthane | 0,20 % |
| Carbomer | 0,15 % |
| Neutralisant | qs. |
| Conservateurs | qs. |
| Parfum, Colorants | qs. |
| Eau | qsp 100,00 % |

**Exemple 2 : Emulsion-gel pour le visage, anti-rides**

| | |
|---|---|
| Glycérine | 5,00 % |
| Caprylic/capric/Succinic triglycérides | 3,00 % |
| Extrait E2 de l'exemple 1.2 | 2,00 % |
| Méthoxycinnamate d'octyl | 1,00 % |
| Polymère réticulé : Acrylates/ acrylate d'alkyle en $C_{10-30}$ | 0,50 % |
| Hydrolysat de protéine de blé | 0,50 % |
| Diméthicone copolyol | 0,50 % |
| Neutralisant | qs. |
| Conservateurs | qs. |
| Parfum, Colorants | qs. |
| Eau | qsp 100,00 % |

**Exemple 3: Emulsion raffermissante pour le corps**

| | |
|---|---|
| Palmitate d'octyl | 7,00 % |
| Tricaprylate/caprate de glycérol | 3,00 % |
| Octanoate d'octyl | 2,00 % |
| Phényl triméthicone | 2,00 % |
| Glycérine | 2,00 % |
| Acide stéarique | 1,00 % |
| Stéarate de sorbitan, 20 OE | 0,90 % |
| Alcool cétylique | 0,50 % |
| Alcool stéarylique | 0,50 % |
| Extrait E2 de l'exemple 1.2 | 0,50 % |
| Carbomer | 0,40 % |
| Gomme Xanthane | 0,20 % |
| Stéarate de sorbitan | 0,10 % |
| Neutralisant | qs. |
| Conservateurs | qs. |
| Parfum, Colorants | qs. |
| Eau | qsp 100,00 % |

**Exemple 4 : Emulsion-gel pour le visage, anti-rides**

| | |
|---|---|
| Glycérine | 5,00 % |
| Caprylidcapric/Succinic triglycérides | 3,00 % |

(suite)

| | |
|---|---|
| Méthoxycinnamate d'octyl | 1,00 % |
| Polymère réticulé : Acrylates/ acrylate d'alkyle en $C_{10-30}$ | 0,50 % |
| Hydrolysat de protéine de blé | 0,50 % |
| Diméthicone copolyol | 0,50 % |
| Extrait E2 de l'exemple 1.2. | 0,01 % |
| Neutralisant | qs. |
| Conservateurs | qs. |
| Parfum, Colorants | qs. |
| Eau | qsp 100,00 % |

## Schéma 1

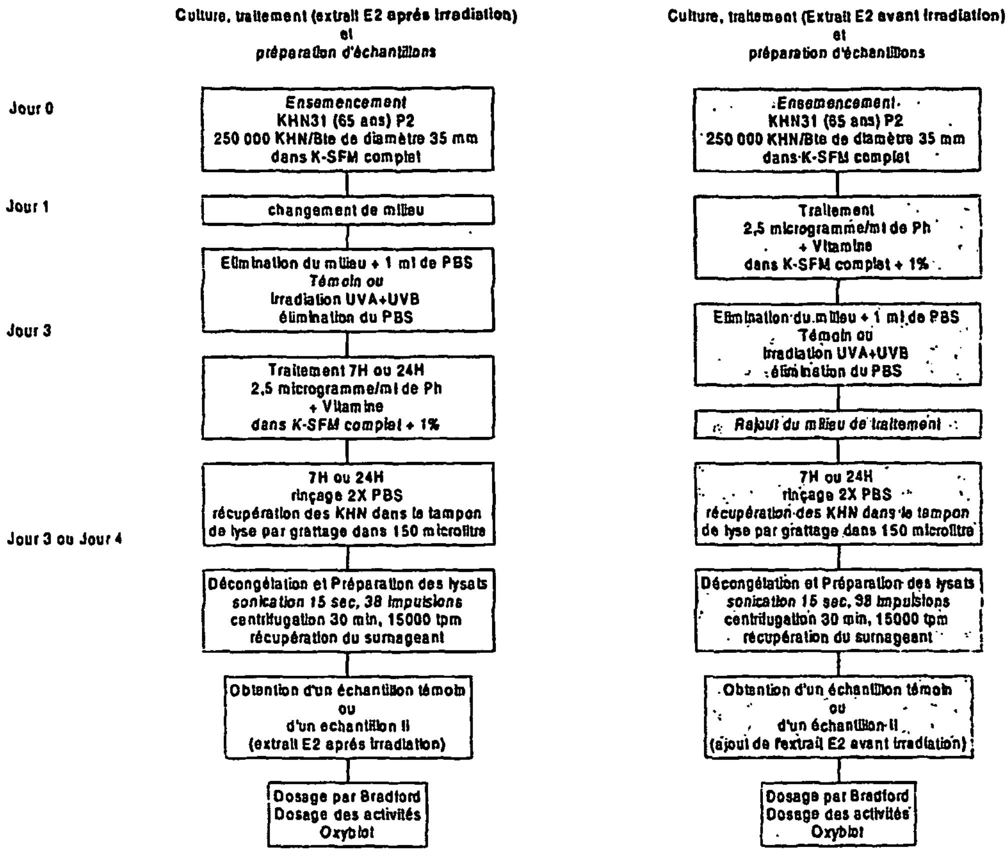

**Tableau 8** (Mesure d'activité LLVY du protéasome issu de KHN après traitement avec l'extrait E2

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| Témoin | 20 µl<br>20 µl<br>20 µl<br>20 µl | 1,540<br>1,373<br>1,599<br>1,488 | 1,500 ± 0,096 | 480,00<br>418,80<br>551,04<br>431,03 | 90,96<br>89,02<br>100,5<br>7 84,55 | 91,27 ± 6,75 | | |
| Extrait E2 5µg/ml | 20 µl<br>20 µl<br>20 µl<br>20 µl | 1,521<br>1,544<br>1,535<br>1,599 | 1,550 ± 0,034 | 555,11<br>553,17<br>515,22<br>545,13 | 106,5<br>3<br>104,6<br>0<br>97,98<br>99,49 | 102,15 ± 4,07 | 0,032<br>9 S | |
| **Significatif(S) si la valeur de p≤0,05 / 0,2919 X xUF/min pour 15 µl de prise d'essai | | | | | | | | |

**Tableau 9 :** (Mesure d'activité LLE du protéasome issu de KHN après traitement avec l'extrait E2

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| Témoin | 20 µl<br>20 µl<br>20 µl<br>20 µl | 1,540<br>1,373<br>1,599<br>0,096<br>1,488 | 1,500 ± | 357,29<br>340,57<br>425,01<br>322,79 | 191,97<br>205,24<br>219,92<br>179,52 | 199,16 ± 17,37 | | |
| Extrait E2 5µg/ml | 20 µl<br>20 µl<br>20 µl<br>20 µl | 1,521<br>1,544<br>1,535<br>1,599 | 1,550 ± 0,034 | 424,63<br>425,65<br>345,36<br>387,58 | 231,03<br>228,20<br>186,20<br>200,55 | 211,50 ± 21,76 | 0,409<br>7 S | |
| **Significatif(S) si la valeur de p≤0,05 / 1,3794 X xUF/min pour 15 µl de prise d'essai | | | | | | | | |

**Tableau 10** : (Mesure d'activité LSTR du protéasome issu de KHN après traitement avec l'extrait E2

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| Témoin | 20 µl<br>20 µl<br>20 µl<br>20 µl | 1,540<br>1,373<br>1,599<br>1,488 | 1,500 ± 0,096 | 951,85<br>885,25<br>1104,90<br>864,47 | 204,36<br>213,17<br>228,45<br>192,11 | 209,53 ± 15,29 | | |

(suite)

| Echantillon | | Protéine en µg/µl moyenne écart type | | Vitesse en UF/min | Activité en pmol/min/mg de protéine moyenne écart type | | Test t p** /tém UV- | Test t p** /tém UV+ |
|---|---|---|---|---|---|---|---|---|
| | 20 µl | 1,521 | | 1141,60 | 248,19 | | | |
| Extrait | 20 µl | 1,544 | | 1142,90 | 244,84 | | | |
| E2 | 20 µl | 1,535 | 1,550 ± 0,034 | 1092,00 | 235,26 | 239,01 ± 9,29 | 0,0165 | |
| 5µg/ml | 20 µl | 1,599 | | 1101,50 | 227,75 | | S | |
| **Significatif(S) si la valeur de p≤0,05 / 0,3307X xUF/min pour 35 µl de prise d'essai | | | | | | | | |

**Tableau 11:** (Mesure de l'activité LLVY du protéasome des KHN traités préalablement avec l'extrait E2, 24 h avant irradiation UVA et UVB.)

| Echantillon | | Protéine en µg/µl moyenne écart type | | Vitesse en UF/min | Activité en pmol/min/mg de protéine moyenne écart type | | Test t p** /tém UV- | Test t p** /tém UV+ |
|---|---|---|---|---|---|---|---|---|
| | 20 µl | 1,038 | | 260,82 | 54,99 | | | |
| | 20 µl | 1,030 | 1,153 ± 0,141 | 363,22 | 77,22 | | | |
| Témoin | 20 µl | 1,311 | | 351,97 | 58,76 | 63,66 ± 10,19 | | |
| | 20 µl | 1,232 | | 392,97 | 69,80 | | | |
| Témoin | 20 µl | 1,407 | | 253,25 | 39,39 | 37,08 ± 3,27 | 0,0224 | |
| + UV | 20 µl | 1,444 | 1,426 ± 0,026 | 229,26 | 34,76 | | S | |
| | 20 µl | 0,998 | | 514,70 | 112,94 | | | |
| Extrait | 20 µl | 1,121 | 1,203 ± 0,173 | 458,06 | 89,41 | | | |
| E2 | 20 µl | 1,326 | | 474,78 | 78,36 | 96,90 ± 15,88 | 0,0152 | |
| 5µg/ml | 20 µl | 1,365 | | 666,44 | 106,90 | | S | |
| | 20 µl | 1,264 | | 329,56 | 57,06 | | | |
| ExtraitE | 20 µl | 1,322 | 1,255 ± 0,073 | 283,15 | 46,88 | | | |
| 2 + UV | 20 µl | 1,151 | | 369,35 | 70,23 | 60,53 ± 10,76 | 0,5522 | 0,0458 |
| | 20 µl | 1,284 | | 398,45 | 67,95 | | NS | S |
| **Significatif(S) si la valeur de p≤0,05 / 0,2189X xUF/min pour 20µl de prise d'essai | | | | | | | | |

**Tableau 12:** (Mesure de l'activité LLE du protéasome des KHN traités préalablement avec l'extrait E2, 24 h avant irradiation UVA et UVB)

| Echantillon | | Protéine en µg/µl moyenne écart type | Vitesse en UF/min | Activité en pmol/min/mg de protéine moyenne écart type | | Test t p** /tém UV- | Test t p** /tém UV+ |
|---|---|---|---|---|---|---|---|
| | 20 µl | 1,038 | 256,79 | 255,90 | | | |

(suite)

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| Témoin | 20 µl | 1,030 | 1,153 ± 0,141 | 377,91 | 379,75 | | | |
| | 20 µl | 1,311 | | 373,48 | 294,67 | 310,11 ± 53,70 | | |
| | 20 µl | 1,232 | | 403,79 | 338,98 | | | |
| Témoin + UV | 20 µl | 1,407 | | 271,40 | 199,51 | 192,42 ± 10,02 | 0,0368 S | |
| | 20 µl | 1,444 | 1,426 ± 0,026 | 258,62 | 185,34 | | | |
| Extrait E2 5µg/ml | 20 µl | 0,998 | | 532,71 | 552,47 | | | |
| | 20 µl | 1,121 | 1,203 + 0,173 | 491,76 | 453,70 | | | |
| | 20 µl | 1,326 | | 595,53 | 464,55 | 493,35 ± 44,66 | 0,0024 S | |
| | 20 µg | 1,365 | | 663,05 | 502,67 | | | |
| Extrait E2 + UV | 20 µl | 1,264 | | 335,07 | 274,17 | | | |
| | 20 µl | 1,322 | 1,255 ± 0,073 | 315,22 | 246,69 | | | |
| | 20 µl | 1,151 | | 381,19 | 342,55 | 295,46 ± 43,12 | 0,5489 | 0,0342 |
| | 20 µl | 1,284 | | 395,07 | 318,43 | | NS | S |
| **Significatif(S) si la valeur de p≤0,05 / 1,0346X xUF/min pour 20µl de prise d'essai | | | | | | | | |

**Tableau 13**: (Mesure de l'activité LSTR du protéasome issu des KHN, traités préalablement avec l'extrait E2 , 24 h avant irradiation UVA et UVB)

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| Témoin | 50µl | 1,038 | | 577,08 | 128,68 | | | |
| | 50µl | 1,030 | 1,153 ± 0,141 | 789,09 | 177,42 | | | |
| | 50µl | 1,311 | | 789,96 | 139,46 | 148,52 ± 21,51 | | |
| | 50µl | 1,232 | | 844,41 | 158,62 | | | |
| Témoin + UV | 50µl | 1,407 | | 564,46 | 92,85 | 87,33 ± 7,80 | 0,0181 | |
| | 50µl | 1,444 | 1,426 ± 0,026 | 510,24 | 81,82 | | S | |
| Extrait E2 5µg/ml | 50µl | 0,998 | | 1079,40 | 250,48 | | | |
| | 50µl | 1,121 | 1,203 ± 0,173 | 1001,40 | 206,73 | | | |
| | 50µl | 1,326 | | 1110,60 | 193,85 | 216,29 ± 24,28 | 0,0069 | |

(suite)

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| | 50µl | 1,365 | | 1262,10 | 214,10 | | S | |
| | 50µl | 1,264 | | 682,00 | 124,87 | | | |
| Extrait E2 | 50µl | 1,322 | 1,255 ± 0,073 | | | | | |
| + UV | 50µl | 1,151 | | 771,19 | 155,07 | 140,49 ± 15,13 | 0,5041 | 0,021 |
| | 50µl | 1,284 | | 784,76 | 141,53 | | NS | 4 S |
| **Significatif(S) si la valeur de p≤0,05 / 0,2315X xUF/min pour 50µl de prise d'essai | | | | | | | | |

**Tableau 14:** (Mesure de l'activité LLVY du protéasome des KHN 7 h après irradiation UVA + UVB puis traitement avec l'extrait E2)

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| | 20 µl | 0,320 | | 532,43 | | | | |
| | 20 µl | 0,563 | 0,697 ± 0,321 | 475,60 | 184,89 | | | |
| Témoin | 20 µl | 0,857 | | 448,06 | 114,47 | 142,53 ± 37,32 | | |
| | 20 µl | 1,050 | | 614,88 | 128,24 | | | |
| | 20 µl | 0,967 | | 219,93 | 49,81 | | | |
| Témoin + | 20 µl | 1,067 | 1,105 ± 0,172 | 211,11 | 43,29 | | | |
| UV | 20 µl | 1,032 | | 255,43 | 54,19 | 51,37 ± 6,38 | 0,0043 S | |
| | 20 µl | 1,355 | | 360,17 | 58,18 | | | |
| | 20 µl | 0,466 | | 793,98 | | | | |
| Extrait E2 | 20 µl | 0,486 | 0,677 ± 0,237 | 703,83 | | | | |
| 5µg/ml | 20 µl | 0,815 | | 752,11 | 201,93 | 189,35 ± 17,79 | 0,2090 | |
| | 20 µl | 0,940 | | 758,93 | 176,77 | | NS | |
| | 20 µl | 1,257 | | 846,29 | 147,35 | | | |
| Extrait E2 | 20 µl | 1,334 | 1,248 ± 0,100 | 806,37 | 132,29 | | | |
| + UV | 20 µl | 1,106 | | 849,40 | 168,13 | 163,40 ± 31,88 | 0,4600 | 0,0005 S |
| | 20 µl | 1,296 | | 1218,50 | 205,84 | | NS | |
| **Significatif(S) si la valeur de p≤0,05 / 0,2189X xUF/min pour 20µl de prise d'essai | | | | | | | | |

**Tableau 15**: (Mesure de l'activité LSTR du protéasome issu de KHN 7 h après irradiation UVA et UVB puis traitement avec l'extrait E2 l'invention

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| Témoin | 40 µl | 0,320 | 0,697 ± 0,321 | 1115,40 | | 318,86 ± 2,75 | | |
| | 40 µl | 0,563 | | 966,14 | | | | |
| | 40 µl | 0,857 | | 821,07 | 316,91 | | | |
| | 40 µl | 1,050 | | 1163,50 | 320,80 | | | |
| Témoin + UV | 40 µl | 0,967 | 1,105 ± 0,172 | 463,17 | 158,48 | 161,03 ± 7,18 | 0,0000 S | |
| | 40 µl | 1,067 | | 498,85 | 154,55 | | | |
| | 40 µl | 1,032 | | 534,34 | 171,26 | | | |
| | 40 µl | 1,355 | | 748,43 | 159,84 | | | |
| Extrait E2 5µg/ml | 40 µl | 0,466 | 0,677 ± 0,237 | 1354,50 | | 397,08 ± 24,86 | 0,0475 S | |
| | 40 µl | 0,486 | | 1383,60 | | | | |
| | 40 µl | 0,815 | | 1168,20 | 414,67 | | | |
| | 40 µl | 0,940 | | 1232,40 | 379,50 | | | |
| Extrait E2 + UV | 40 µl | 1,257 | 1,248 ± 0,100 | 1326,90 | 305,44 | 314,44 ± 37,30 | 0,8824 NS | 0,0002 S |
| | 40 µl | 1,334 | | 1228,40 | 266,43 | | | |
| | 40 µl | 1,106 | | 1276,10 | 333,94 | | | |
| | 40 µl | 1,296 | | 1575,90 | 351,96 | | | |
| **Significatif(S) si la valeur de p≤0,05 / 0,2894X xUF/min pour 40µl de prise d'essai | | | | | | | | |

**Tableau 16**: (Mesure de l'activité LLE du protéasome issu de KHN 7 h après irradiation UVA et UVB puis traitement avec l'extrait E2)

| Echantillon | | Protéine en µg/µl moyenne écart type | | Vitesse en UF/min | Activité en pmol/min/mg de protéine moyenne écart type | | Test t p** | |
|---|---|---|---|---|---|---|---|---|
| | | | | | | | /tém UV- | /tém UV+ |
| Témoin | 20 µl | 0,320 | 0,697 ± 0,321 | 446,80 | | 474,49 ± 3,21 | | |
| | 20 µl | 0,563 | | 429,01 | | | | |
| | 20 µl | 0,857 | | 391,07 | 472,22 | | | |
| | 20 µl | 1,050 | | 483,67 | 476,76 | | | |
| Témoin + UV | 20 µl | 0,967 | 1,105 ± 0,172 | 183,97 | 196,93 | 204,81 ± 26,39 | 0,0002 S | |
| | 20 µl | 1,067 | | 177,14 | 171,70 | | | |
| | 20 µl | 1,032 | | 218,01 | 218,60 | | | |
| | 20 µl | 1,355 | | 303,89 | 232,02 | | | |

(suite)

| Echantillon | | Protéine en µg/µl moyenne écart type | | Vitesse en UF/min | Activité en pmol/min/mg de protéine moyenne écart type | | Test t p** /tém UV- | Test t p** /tém UV+ |
|---|---|---|---|---|---|---|---|---|
| Extrait E2 5µg/ml | 20 µl | 0,466 | 0,677 ± 0,237 | 588,98 | | 671;61 ± 123,83 | 0,1533 NS | |
| | 20 µl | 0,486 | | 542,38 | | | | |
| | 20 µl | 0,815 | | 598,25 | 759,17 | | | |
| | 20 µl | 0,940 | | 530,53 | 584,05 | | | |
| Extrait E2 + UV | 20 µl | 1,257 | 1,248 ± 0,100 | 576,40 | 474,34 | 534,53 ± 75,84 | 0,3509 S | 0,0002 S |
| | 20 µl | 1,334 | | 598,01 | 463,69 | | | |
| | 20 µl | 1,106 | | 637,05 | 595,98 | | | |
| | 20 µl | 1,296 | | 756,61 | 604,10 | | | |
| **Significatif(S) si la valeur de p≤0,05 / 1,0346X xUF/min pour 20µl de prise d'essai | | | | | | | | |

**Revendications**

1. Utilisation d'un extrait de l'algue Phaeodactylum, en particulier de l'algue Phaeodactylum tricornutum, comme agent cosmétique favorisant l'activité du protéasome des cellules de la peau, en particulier des kératinocytes, des fibroblastes ou des mélanocytes, de préférence humains.

2. Composition comprenant un extrait de l'algue Phaeodactylum, en particulier Phaeodactylum tricornutum, pour protéger la peau contre les effets néfastes d'une exposition aux UV, ou prévenir et/ou retarder l'apparition des effets du vieillissement cutané.

3. Utilisation selon la revendication 1 ou composition selon la revendication 2, **caractérisée en ce que** ledit extrait de l'algue est utilisé dans une composition à une concentration de 0,01 % à 10 % environ par rapport au poids total de la composition finale.

4. Utilisation ou composition selon l'une des revendications 1 à 3, **caractérisée en ce que** l'extrait précité a été obtenu par extraction au moyen d'un solvant d'extraction polaire et/ou un solvant d'extraction apolaire.

5. Utilisation ou composition selon la revendication 4 **caractérisée en ce que** le ou les solvants d'extraction sont un alcool en $C_1$-$C_6$ ou un mélange hydroalcoolique, un polyalcool en $C_2$-$C_6$ tel que l'éthylène glycol, un solvant chloré tel que le chloroforme ou le dichlorométhane, un ester en $C_3$-$C_6$ d'acide organique tel qu'acétate d'éthyle, un alcane en $C_6$-$C_{10}$ tel que l'heptane, un éther en $C_5$-$C_8$ comme par exemple le diisopropyle éther.

6. Utilisation ou composition selon la revendication 5, **caractérisée en ce que** l'extrait précité est obtenu par extraction de l'algue avec un alcool ou un mélange eau/alcool éventuellement alcalinisé, ledit alcool étant choisi dans le groupe constitué de l'isopropanol, de l'éthanol, et du méthanol.

7. Utilisation ou composition selon la revendication 6, **caractérisée en ce que** l'extrait précité est obtenu par extraction de l'algue avec de l'isopropanol.

8. Utilisation ou composition selon la revendication 6, **caractérisée en ce que** l'extrait précité est obtenu par l'extraction de l'algue avec de l'éthanol.

9. Utilisation ou composition selon l'une des revendications 4 à 8, **caractérisée en ce que**, préalablement à son extraction, l'algue est congelée, de préférence la congélation étant réalisée à une température située entre -40°C et -20°C environ et pendant une durée comprise de préférence entre 1 et 7 jours environ, puis mise en contact avec

le solvant d'extraction.

**10.** Utilisation ou composition selon la revendication 9, **caractérisée en ce que** l'algue congelée est plongée directement dans le solvant d'extraction chauffé.

**11.** Utilisation ou composition selon l'une des revendications 4 à 10, **caractérisée en ce que**, avant toute opération d'extraction, on réalise une macération de l'algue dans le solvant d'extraction à température ambiante

**12.** Utilisation ou composition selon la revendication 11, **caractérisée en ce qu'**on réalise une macération de l'algue pendant une durée comprise entre 5 minutes et 80 minutes, environ, et de préférence encore, pendant une durée comprise entre 20 minutes et 40 minutes environ.

**13.** Utilisation ou composition selon l'une des revendications 4 à 12, **caractérisée en ce que** l'extraction est réalisée sous reflux.

**14.** Utilisation ou composition selon l'une des revendications 4 à 13, **caractérisée en ce que** l'extraction est réalisée sous atmosphère inerte, de préférence sous atmosphère saturée en azote.

**15.** Utilisation ou composition selon l'une des revendications 6 à 14, **caractérisée en ce que** l'extrait d'algue précité est obtenu après la succession des étapes suivantes :

a) l'algue est congelée selon la revendication 9, et ensuite plongée dans le solvant d'extraction selon la revendication 10, ledit solvant d'extraction étant un solvant alcoolique ou hydro-alcoolique défini aux revendications 6 à 8,
b) une macération de l'algue est effectuée suivant la revendication 11 ou 12,
c) le solvant d'extraction est alcalinisé jusqu'à un pH compris entre 10 et 14, de préférence à un pH égal à 13, par exemple avec une solution aqueuse d'hydroxyde de sodium ou avec une solution aqueuse d'hydroxyde de potassium,
d) les insolubles sont éliminés de la phase alcoolique ou hydroalcoolique,
e) de l'eau distillée est ajoutée à la phase alcoolique ou hydroalcoolique,
f) la solution hydro-alcoolique ainsi obtenue est lavée par un procédé liquide/liquide avec un solvant apolaire non miscible avec la phase alcoolique ou hydro-alcoolique, tel que par exemple l'heptane, l'hexane ou le cyclohexane,
g) la phase contenant le solvant apolaire est éliminée,
h) la phase hydro-alcoolique récupérée après élimination de la phase contenant le solvant apolaire, est acidifiée jusqu'à un pH compris entre 1 et 3, de préférence à un pH égal à 2, par exemple avec une solution aqueuse d'acide sulfurique ou avec une solution aqueuse d'acide chlorhydrique,
i) la solution obtenue après acidification subit une extraction liquide-liquide avec un solvant apolaire non miscible avec la phase alcoolique ou hydro-alcoolique, tel que par exemple l'heptane, l'hexane ou le cyclohexane,
j) la phase hydro-alcoolique est ensuite éliminée,
k) la phase contenant le solvant apolaire récupérée après élimination de la phase hydro-alcoolique subit une évaporation afin d'obtenir une huile exempte de solvant apolaire, cette huile étant l'extrait recherché.

**16.** Utilisation ou composition selon l'une des revendications 4 à 15, **caractérisée en ce que** la quantité de solvant d'extraction utilisée est comprise entre 0,1 litre et 20 litres environ, de préférence comprise entre 2 litres et 10 litres environ, pour une quantité de 100 g d'algue, exprimée en poids sec.

**17.** Utilisation ou composition selon l'une des revendications 1 à 3, **caractérisée en ce que** ledit extrait de l'algue a été obtenu par extraction au moyen du $CO_2$ supercritique.

**Claims**

**1.** Use of an extract of the alga Phaeodactylum, particularly the alga Phaeodactylum tricornutum, as a cosmetic agent promoting the proteasome activity of skin cells, particularly keratinocytes, fibroblasts or melanocytes, preferably humans.

**2.** Composition comprising an extract of the alga Phaeodactylum, particularly Phaeodactylum tricornutum, for protecting

the skin from the adverse effects of UV exposure or for preventing and/or delaying the appearance of skin ageing effects.

**3.** Use according to claim 1 or composition according to claim 2, **characterized in that** said algal extract is used in a composition in a concentration from about 0.01% to 10%, based on the total weight of the final composition.

**4.** Use or composition according to one of claims 1 to 3, **characterized in that** the extract has been obtained by extraction with a polar extraction solvent and/or an apolar extraction solvent.

**5.** Use or composition according to claim 4, **characterized in that** the extraction solvent or solvents are a $C_1$-$C_6$ alcohol or an aqueous-alcoholic mixture, a $C_2$-$C_6$ polyhydric alcohol such as ethylene glycol, a chlorinated solvent such as chloroform or dichloromethane, a $C_3$-$C_6$ ester of an organic acid, such as ethyl acetate, a $C_6$-$C_{10}$ alkane such as heptane, or a $C_5$-$C_8$ ether such as diisopropyl ether.

**6.** Use or composition according to claim 5, **characterized in that** the extract is obtained by extraction of the alga with an alcohol or a water/alcohol mixture which has optionally been rendered alkaline, said alcohol being selected from the group comprising isopropanol, ethanol and methanol.

**7.** Use or composition according to claim 6, **characterized in that** the aforementioned extract is obtained by extraction of the alga with isopropanol.

**8.** Use or composition according to claim 6, **characterized in that** the extract is obtained by extraction of the alga with ethanol.

**9.** Use or composition according to one of claims 4 to 8, **characterized in that** the alga is frozen before it is extracted, the freezing preferably being effected at a temperature of between about -40°C and -20°C and for a period preferably of between about 1 and 7 days, after which it is brought into contact with the extraction solvent.

**10.** Use or composition according to claim 9, **characterized in that** the frozen alga is immersed directly in the heated extraction solvent.

**11.** Use or composition according to one of claims 4 to 10, **characterized in that**, before any extraction operation, the alga is macerated in the extraction solvent at room temperature.

**12.** Use or composition according to claim 11, **characterized in that** the alga is macerated for a period of between about 5 minutes and 80 minutes, particularly preferably for a period of between about 20 minutes and 40 minutes.

**13.** Use or composition according to one of claims 4 to 12, **characterized in that** the extraction is carried out under reflux.

**14.** Use or composition according to one of claims 4 to 13, **characterized in that** the extraction is carried out under an inert atmosphere, preferably under a nitrogen-saturated atmosphere.

**15.** Use or composition according to one of claims 6 to 14, **characterized in that** the aforementioned algal extract is obtained after the following series of steps:

a) the alga is frozen according to claim 9 and then immersed in the extraction solvent according to claim 10, said extraction solvent being an alcoholic or aqueous-alcoholic solvent defined in claims 6 to 8,
b) the alga is macerated according to claim 11 or 12,
c) the extraction solvent is rendered alkaline to a pH of between 10 and 14, preferably to a pH of 13, for example with aqueous sodium hydroxide solution or aqueous potassium hydroxide solution,
d) the insoluble materials are removed from the alcoholic or aqueous-alcoholic phase,
e) distilled water is added to the alcoholic or aqueous-alcoholic phase,
f) the resulting aqueous-alcoholic solution is washed by a liquid-liquid process with an apolar solvent that is immiscible with the alcoholic or aqueous-alcoholic phase, for example heptane, hexane or cyclohexane,
g) the phase containing the apolar solvent is removed,
h) the aqueous-alcoholic phase recovered after removal of the phase containing the apolar solvent is acidified to a pH of between 1 and 3, preferably to a pH of 2, for example with aqueous sulfuric acid solution or aqueous hydrochloric acid solution,

i) the solution obtained after acidification is subjected to a liquid-liquid extraction with an apolar solvent that is immiscible with the alcoholic or aqueous-alcoholic phase, for example heptane, hexane or cyclohexane,
j) the aqueous-alcoholic phase is then removed, and
k) the phase containing the apolar solvent, recovered after removal of the aqueous-alcoholic phase, is subjected to evaporation to give an oil free of apolar solvent, this oil being the desired extract.

**16.** Use or composition according to one of claims 4 to 15, **characterized in that** the amount of extraction solvent used is between about 0.1 liter and 20 liters, preferably between about 2 liters and 10 liters, per 100 g of alga, expressed by dry weight.

**17.** Use or composition according to one of claims 1 to 3, **characterized in that** said algal extract has been obtained by extraction with supercritical $CO_2$.

**Patentansprüche**

**1.** Verwendung eines Extraktes aus der Alge Phaeodactylum, insbesondere aus der Alge Phaeodactylum tricornutum, als kosmetischer Wirkstoff, der die Aktivität des Proteasoms der Hautzellen, insbesondere der Keratinozyten, der Fibroblasten oder der Melanozyten, vorzugsweise menschlichen, begünstigt.

**2.** Zusammensetzung, die einen Extrakt aus der Alge Phaeodactylum, insbesondere Phaeodactylum tricornutum umfaßt, um die Haut vor den schädlichen Auswirkungen einer UV-Exposition zu schützen oder um dem Auftreten der Folgen der Hautalterung vorzubeugen und/oder dieses zu verzögern.

**3.** Verwendung nach Anspruch 1 oder Zusammensetzung nach Anspruch 2, **dadurch gekennzeichnet, daß** der Extrakt aus der Alge in einer Zusammensetzung in einer Konzentration von etwa 0,01 % bis 10 % bezogen auf das Gesamtgewicht der Endzusammensetzung verwendet wird.

**4.** Verwendung oder Zusammensetzung nach einem der Ansprüche 1 bis 3, **dadurch gekennzeichnet, daß** der vorgenannte Extrakt durch Extraktion mit Hilfe eines polaren Extraktionslösungsmittels und/oder eines unpolaren Extraktionslösungsmittels gewonnen wurde.

**5.** Verwendung oder Zusammensetzung nach Anspruch 4, **dadurch gekennzeichnet, daß** das oder die Extraktionslösungsmittel ein $C_1$-$C_6$-Alkohol oder ein hydroalkoholisches Gemisch, ein $C_2$-$C_6$-Polyalkohol, wie Ethylenglykol, ein chloriertes Lösungsmittel, wie Chloroform oder Dichlormethan, ein $C_3$-$C_6$-Ester einer organischen Säure, wie Ethylacetat, ein $C_6$-$C_{10}$-Alkan, wie Heptan, ein $C_5$-$C_8$-Ether, wie zum Beispiel Diisopropylether sind.

**6.** Verwendung oder Zusammensetzung nach Anspruch 5, **dadurch gekennzeichnet, daß** der vorgenannte Extrakt durch Extraktion der Alge mit einem Alkohol oder einem Wasser/Alkohol-Gemisch, eventuell alkalisiert, gewonnen wird, wobei der Alkohol aus der Gruppe bestehend aus Isopropanol, Ethanol und Methanol ausgewählt ist.

**7.** Verwendung oder Zusammensetzung nach Anspruch 6, **dadurch gekennzeichnet, daß** der vorgenannte Extrakt durch Extraktion der Alge mit Isopropanol gewonnen wird.

**8.** Verwendung oder Zusammensetzung nach Anspruch 6, **dadurch gekennzeichnet, daß** der vorgenannte Extrakt durch Extraktion der Alge mit Ethanol gewonnen wird.

**9.** Verwendung oder Zusammensetzung nach einem der Ansprüche 4 bis 8, **dadurch gekennzeichnet, daß** die Alge vor ihrer Extraktion tiefgefroren wird, wobei vorzugsweise das Tiefgefrieren bei einer Temperatur im Bereich zwischen etwa - 40 °C und - 20 °C und über einen Zeitraum von vorzugsweise etwa 1 bis 7 Tagen durchgeführt wird, anschließend mit dem Extraktionslösungsmittel in Kontakt gebracht wird.

**10.** Verwendung oder Zusammensetzung nach Anspruch 9, **dadurch gekennzeichnet, daß** die tiefgefrorene Alge direkt in das erhitzte Extraktionslösungsmittel getaucht wird.

**11.** Verwendung oder Zusammensetzung nach einem der Ansprüche 4 bis 10, **dadurch gekennzeichnet, daß** vor jedem Extraktionsschritt eine Mazeration der Alge in dem Extraktionslösungsmittel bei Raumtemperatur durchgeführt wird.

**12.** Verwendung oder Zusammensetzung nach Anspruch 11, **dadurch gekennzeichnet, daß** eine Mazeration der Alge über eine Zeitdauer von etwa 5 Minuten bis 80 Minuten und weiterhin vorzugsweise über eine Zeitdauer zwischen etwa 20 Minuten und 40 Minuten durchgeführt wird.

**13.** Verwendung oder Zusammensetzung nach einem der Ansprüche 4 bis 12, **dadurch gekennzeichnet, daß** die Extraktion unter Rückfluß durchgeführt wird.

**14.** Verwendung oder Zusammensetzung nach einem der Ansprüche 4 bis 13, **dadurch gekennzeichnet, daß** die Extraktion unter inerter Atmosphäre, vorzugsweise unter stickstoffgesättigter Atmosphäre durchgeführt wird.

**15.** Verwendung oder Zusammensetzung nach einem der Ansprüche 6 bis 14, **dadurch gekennzeichnet, daß** der vorgenannte Algenextrakt nach der Aufeinanderfolge der folgenden Schritte erhalten wird:

a) die Alge wird gemäß Anspruch 9 tiefgefroren und anschließend gemäß Anspruch 10 in das Extraktionslösungsmittel getaucht, wobei das Extraktionslösungsmittel ein in den Ansprüchen 6 bis 8 definiertes alkoholisches oder hydroalkoholisches Lösungsmittel ist,
b) es wird eine Mazeration der Alge gemäß Anspruch 11 oder 12 durchgeführt,
c) das Extraktionslösungsmittel wird bis zu einem pH-Wert zwischen 10 und 14, vorzugsweise bis zu einem pH-Wert gleich 13 alkalisiert, beispielsweise mit einer wäßrigen Natriumhydroxid-Lösung oder mit einer wäßrigen Kaliumhydroxid-Lösung,
d) die unlöslichen Stoffe werden aus der alkoholischen oder hydroalkoholischen Phase entfernt,
e) der alkoholischen oder hydroalkoholischen Phase wird destilliertes Wasser zugegeben,
f) die auf diese Weise erhaltene hydroalkoholische Lösung wird mittels eines Flüssig-Flüssig-Verfahrens mit einem unpolaren Lösungsmittel, das mit der alkoholischen oder hydroalkoholischen Phase nicht mischbar ist, wie zum Beispiel Heptan, Hexan oder Cyclohexan, gewaschen,
g) die das unpolare Lösungsmittel enthaltende Phase wird entfernt,
h) die hydroalkoholische Phase, die nach Entfernen der das unpolare Lösungsmittel enthaltenden Phase gewonnen wird, wird bis zu einem pH-Wert zwischen 1 und 3, vorzugsweise bis zu einem pH-Wert gleich 2 angesäuert, beispielweise mit einer wäßrigen Schwefelsäurelösung oder mit einer wäßrigen Salzsäurelösung,
i) die nach dem Ansäuern erhaltene Lösung wird einer Flüssig-Flüssig-Extraktion mit einem unpolaren Lösungsmittel, das mit der alkoholischen oder hydroalkoholischen Phase nicht mischbar ist, wie zum Beispiel Heptan, Hexan oder Cyclohexan, unterzogen,
j) die hydroalkoholische Phase wird anschließend entfernt,
k) die das unpolare Lösungsmittel enthaltende Phase, die nach Entfernen der hydroalkoholischen Phase gewonnen wird, wird einem Verdampfen unterzogen, um ein Öl ohne unpolares Lösungsmittel zu erhalten, wobei dieses Öl der gewünschte Extrakt ist.

**16.** Verwendung oder Zusammensetzung nach einem der Ansprüche 4 bis 15, **dadurch gekennzeichnet, daß** die verwendete Extraktionslösungsmittelmenge zwischen etwa 0,1 Liter und 20 Litern, vorzugsweise zwischen etwa 2 Litern und 10 Litern, bei einer Menge von 100 g Alge, ausgedrückt in Trockengewicht, liegt.

**17.** Verwendung oder Zusammensetzung nach einem der Ansprüche 1 bis 3, **dadurch gekennzeichnet, daß** der Extrakt aus der Alge durch Extraktion mit Hilfe von superkritischem $CO_2$ gewonnen wurde.

FIG.1

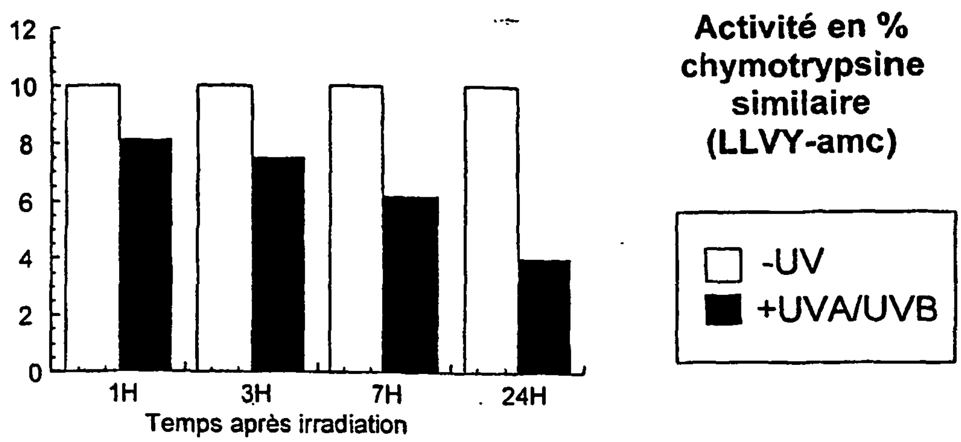


FIG.2

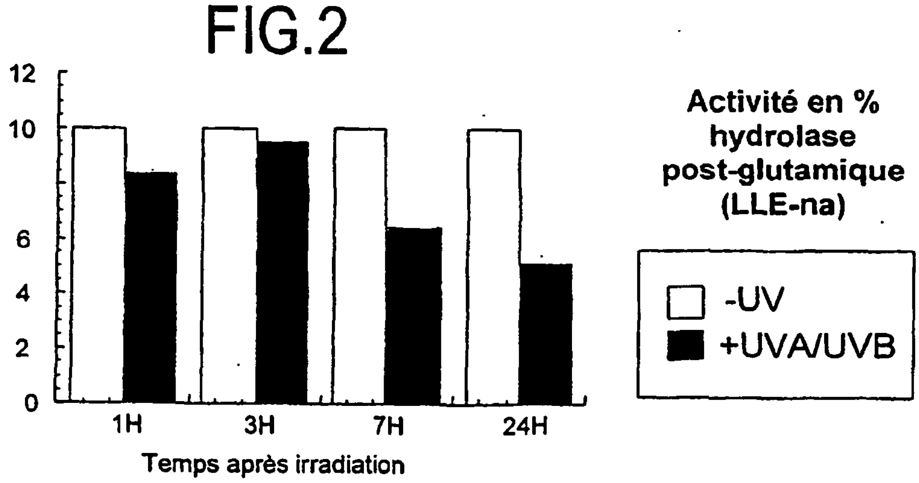


FIG.3

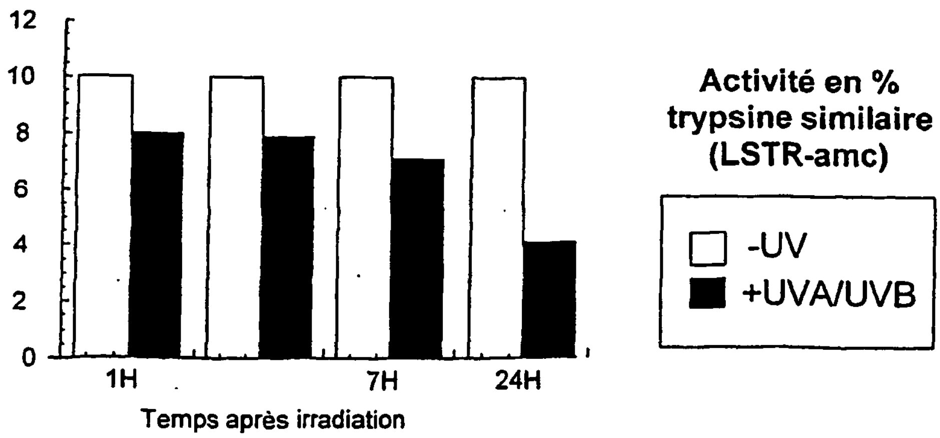

Détection par oxyblot des protéines oxydées
dans des échantillons de KHN irradiés

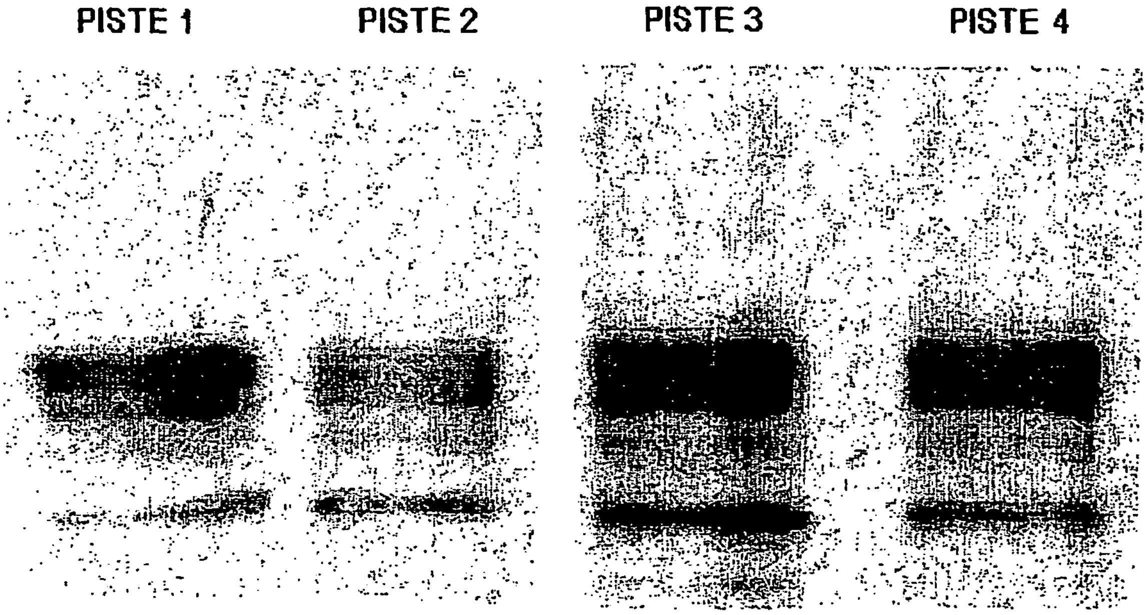


1 Solution témoin
2 Témoin + Phaeodactylum ( échantillon de type I1 )
3 Témoin + UV ( échantillon de type I2 )
4 Témoin + Phaeodactylum + UV ( échantillon de type I3 )

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 07224278 A
- JP 59215386 A
- JP 59204128 A
- EP 0646647 A
- EP 0775449 A


### Littérature non-brevet citée dans la description


- **Friguet B. et al.** *Ann N.Y. Acad. Sci.,* 2000, vol. 908, 143-54
- **Friguet et al.** *Journal of Gerontol. Biol. Sci.,* 2000, vol. 55A (5), B220-B227
- *Patent Abstracts of Japan,* vol. 009 (071
- **Leammli U. K.** Cleavage of structural protéin during the assembly of the head of the bacteriophage T4. *Nature,* 1970, vol. 277, 680-685
- **Bradford, M.** A rapid and sensitive method for the quantitation of microgram quantities of protein utilizing the principle of protein-dye binding. *Anal. Biochem.,* 1976, vol. 72, 248
- **Towbin H. ; Staehelin T. ; Gordon J.** Electrophoretic transfer of proteins from polyacrylamide gels to nitrocellulose sheets : procedure and some applications. *Proc. Natl. Sci., USA,* 1979, vol. 76, 4350-4354